# EUROPEAN PATENT SPECIFICATION

(11) **EP 3 729 441 B1**
(45) Date of publication and mention of the grant of the patent: **13.03.2024**
(21) Application number: 18840007.1
(22) Date of filing: 29.12.2018
(51) Int. Cl.: G16B 20/20, G16B 30/10, G16B 40/00, C12Q 1/6827

(54) **MICROSATELLITE INSTABILITY DETECTION**
ERKENNUNG VON MIKROSATELLITENINSTABILITÄT
DÉTECTION D'INSTABILITÉ DE MICROSATELLITES

(30) Priority: 29.12.2017 US 201762612048 P; 17.07.2018 US 201862699685 P; 28.12.2018 US 201816235901
(43) Date of publication of application: 28.10.2020
(73) Proprietor: Grail, LLC, Menlo Park, CA 94025 (US)
(72) Inventor: HUBBELL, Earl, Menlo Park, CA 94025 (US)
(74) Representative: Schröer, Gernot H.
(86) International application number: PCT/US2018/068066
(87) International publication number: WO 2019/133937

(56) References cited:
- US-A1- 2014 235 456
- GYMREK M ET AL: "lobSTR: A short tandem repeat profiler for personal genomes", GENOME RESEARCH, vol. 22, no. 6, 20 April 2012 (2012-04-20) , pages 1154-1162, XP055560142, US ISSN: 1088-9051, DOI: 10.1101/gr.135780.111
- HAUSE R J ET AL: "Classification and characterization of microsatellite instability across 18 cancer types", NATURE MEDICINE, vol. 22, no. 11, 3 October 2016 (2016-10-03), pages 1342-1350, XP055494424, New York ISSN: 1078-8956, DOI: 10.1038/nm.4191
- BUTLAND S L ET AL: "CAG-encoded polyglutamine length polymorphism in the human genome", BMC GENOMICS, BIOMED CENTRAL, vol. 8, no. 1, 22 May 2007 (2007-05-22), page 126, XP021027940, ISSN: 1471-2164, DOI: 10.1186/1471-2164-8-126
- MISSIRLIS P I ET AL: "Satellog: A database for the identification and prioritization of satellite repeats in disease association studies", BMC BIOINFORMATICS, BIOMED CENTRAL, LONDON, GB, vol. 6, no. 1, 10 June 2005 (2005-06-10), page 145, XP021000738, ISSN: 1471-2105, DOI: 10.1186/1471-2105-6-145
- GYMREK M: "A genomic view of short tandem repeats", CURRENT OPINION IN GENETICS & DEVELOPMENT, vol. 44, 16 February 2017 (2017-02-16), pages 9-16, XP085047725, ISSN: 0959-437X, DOI: 10.1016/J.GDE.2017.01.012

## Description

### BACKGROUND

### 1. FIELD OF ART

This disclosure generally relates to targeted sequencing and more specifically to using both cell free DNA and genomic DNA detecting microsatellite instability.

### 2. DESCRIPTION OF THE RELATED ART

Computational techniques can be used on DNA sequencing data to identify mutations or variants in DNA that may correspond to various types of cancer or other diseases. Further, detecting microsatellite instability (MSI) in those cancers can be informative for method of treatment, likelihood of cure, severity of cancer, progression of cancer, etc. Cancer and MSI diagnosis or prediction may be performed by analyzing a biological sample such as a tissue biopsy or blood drawn from a subject. Methods of detecting MSI in tissue based tumor DNA are known in the art. However, detecting MSI in tumor DNA that originated from circulating cell free DNA (cfDNA) is challenging because circulating tumor DNA (ctDNA) is present at low levels relative to other molecules in cfDNA. Currently, existing methods to identify MSI in tissue based tumor DNA are unreliable in detecting MSI in samples including cfDNA.

US20140235456A1 discloses a method of identifying an increased risk of developing cancer based on an alteration in the microsatellite profile of the subject. However, this prior art does not disclose calculating, for each marker, a marker significance indicating the significance and viability for the marker in detecting MSI.

### SUMMARY

Early detection of cancer in subjects is important as it allows cancer patients (i.e., subjects) a greater chance of surviving the disease. For some cancers, microsatellite instability (MSI) in cell-free DNA can indicate the presence of a cancer which would, otherwise, be hard to detect. As such, subjects can generate a DNA sample for analysis (e.g., a blood sample) to determine a likelihood that MSI exists and, thereby, determine a likelihood that the sample includes cancer.

The present invention concerns a method (400) of detecting a likelihood of cancer in an individual based on MSI, and a corresponding system, as defined in the appended claims.

A processing system is configured to process the sample by separating cfDNA and/or ctDNA, and gDNA (e.g., wbcDNA) from the sample for generating sequence reads that may include MSI. The processing system generates candidate variants from the sequence reads for the cfDNA and/or ctDNA (i.e., variants) and the gDNA (i.e., normals). The processing system compares the variant (i.e., test reads) to the normals (i.e., control reads) to determine if a likelihood that a sample includes MSI.

The processing system determines a likelihood that the sample includes MSI by selecting a set of markers from the sample reads and determining if those markers include MSI associated with cancer. A marker is a one or more reference locations identified from a sequence read or a plurality of sequence reads at a given location of a reference genome. Each marker includes a microsatellite with a microsatellite length indicating the number of times the microsatellite is repeated. For a given marker, the microsatellite length is, generally, substantially similar for control reads, and, generally, dissimilar for test reads. Differences between the markers can be quantified to determine if a given marker is significant in determining MSI. The processing system can filter the markers based on any of the characteristics of the test reads and the control reads (e.g., zygosity, read depth, etc.).

The processing system determines if a marker is significant in determining MSI by calculating, for example, a group of scores for each marker: a viability score, a significance score, an entropy score, and a divergence score. The viability score is a quantification of similarities between test reads and control reads. The significance score is a quantification of the statistical significance of the microsatellite length for a marker. The entropy score is a quantification of a difference in entropy of the marker between test reads and control reads, where entropy is the average uncertainty in microsatellite length. The divergence score is a quantification of the relative difference in the expected observation of microsatellite lengths between test reads and control reads. The processing system determines if a marker is significant based on the scores. Generally, a high viability, significance, entropy, and divergence scores result in a significant marker.

The processing system determines an instability score representing a likelihood that the sample includes MSI based on the determined marker significances. Generally, the likelihood score is a quantification of the relative number of significant to insignificant markers. Based on the instability score, the processing system can determine that a sample includes MSI and inform a method of treatment for the subject.

### BRIEF DESCRIPTION OF DRAWINGS

FIG. 1 is a flowchart of a method for preparing a nucleic acid sample for sequencing according to one embodiment.
FIG. 2 is a block diagram of a processing system for processing sequence reads according to one embodiment.
FIG. 3 is a flowchart of a method for determining variants of sequence reads according to one embodiment.
FIG. 4 is a flow chart of a method for detecting MSI, according to one example embodiment.
FIG. 5A-5B are instability plots illustrating the instability scores for metastatic prostate cancers using a method known in the art and the method of FIG. 4, according to some example embodiments.
FIG. 6 is a flow chart of a method for calculating scores for determining marker significance, according to one example embodiment.
FIGS.7A-7D are significance plots illustrating calculated scores for determining marker significance, according to some example embodiments.
FIG. 8 is a marker plot showing data used to calculate scores for determining marker significance, according to one example embodiment.
FIG. 9A-9B are marker plots for a marker after error correction, the plots comparing a prior art method and the MSI detection method, according to some example embodiments.
FIGS. 9C-9D are characteristic plots comparing the difference in a measure of a characteristic for a marker with a high viability score after error correction, according to some example embodiments.
FIG. 10A-10B are marker plots for a marker after error correction, the plots comparing a prior art method and the MSI detection method according to some example embodiments.
FIGS. 10C-10D are characteristic plots comparing the difference in a measure of a characteristic for a marker with a low viability score after error correction, according to some example embodiments.
FIG. 11A-11B are significance plot for samples including copy number aberration, according to some example embodiments.
FIG. 11C-11D are marker plots for a marker from a sample including copy number aberrations, according to some example embodiments.
FIG. 12A-12B are instability plots illustrating the instability scores for metastatic lung cancer using a method known in the art and the method of FIG. 4, according to some example embodiments.
FIG. 13A- 13D are significance plots for various markers of a metastatic lung cancer sample using the method of FIG. 4, according to some example embodiments.
FIG. 14A-14B are instability plots illustrating the instability scores for metastatic breast cancer using a method known in the art and the method of FIG. 4, according to some example embodiments.
FIG. 15A- 15D are significance plots for various markers of a metastatic breast cancer sample using the method of FIG. 4, according to some example embodiments.
FIG. 16A- 16D are significance plots for various markers of a metastatic prostate cancer sample using the method of FIG. 4, according to some example embodiments.

The figures depict embodiments of the present invention for purposes of illustration only. One skilled in the art will readily recognize from the following discussion that alternative embodiments of the structures and methods illustrated herein may be employed without departing from the principles of the invention described herein.

### DETAILED DESCRIPTION

### I. DEFINITIONS

The term "individual" refers to a human individual. The term "healthy individual" refers to an individual presumed to not have a cancer or disease. The term "subject" refers to an individual who is known to have, or potentially has, a cancer or disease.

The term "sequence reads" refers to nucleotide sequences read from a sample obtained from an individual. Sequence reads can be obtained through various methods known in the art.

The term "read segment" or "read" refers to any nucleotide sequences including sequence reads obtained from an individual and/or nucleotide sequences derived from the initial sequence read from a sample obtained from an individual. For example, a read segment can refer to an aligned sequence read, a collapsed sequence read, or a stitched read. Furthermore, a read segment can refer to an individual nucleotide base, such as a single nucleotide variant.

The term "single nucleotide variant" or "SNV" refers to a substitution of one nucleotide to a different nucleotide at a position (e.g., site) of a nucleotide sequence, e.g., a sequence read from an individual. A substitution from a first nucleobase X to a second nucleobase Y may be denoted as "X>Y." For example, a cytosine to thymine SNV may be denoted as "C>T."

The term "indel" refers to any insertion or deletion of one or more base pairs having a length and a position (which may also be referred to as an anchor position) in a sequence read. An insertion corresponds to a positive length, while a deletion corresponds to a negative length.

The term "mutation" refers to one or more SNVs or indels.

The term "true positive" refers to a mutation that indicates real biology, for example, presence of a potential cancer, disease, or germline mutation in an individual. True positives are not caused by mutations naturally occurring in healthy individuals (e.g., recurrent mutations) or other sources of artifacts such as process errors during assay preparation of nucleic acid samples.

The term "false positive" refers to a mutation incorrectly determined to be a true positive. Generally, false positives may be more likely to occur when processing sequence reads associated with greater mean noise rates or greater uncertainty in noise rates.

The term "cell-free nucleic acid," "cell-free DNA," or "cfDNA" refers to nucleic acid fragments that circulate in an individual's body (e.g., bloodstream) and originate from one or more healthy cells and/or from one or more cancer cells. cfDNA can be obtained from a blood sample.

The term "circulating tumor DNA" or "ctDNA" refers to nucleic acid fragments that originate from tumor cells or other types of cancer cells, which may be released into an individual's bloodstream as result of biological processes such as apoptosis or necrosis of dying cells or actively released by viable tumor cells. In some cases, ctDNA is DNA found in cfDNA.

The term "genomic nucleic acid," "genomic DNA," or "gDNA" refers to nucleic acid including chromosomal DNA that originates from one or more healthy cells. In some cases, white blood cells are assumed to be healthy cells.

The term "white blood cell DNA," or "wbcDNA" refers to nucleic acid including chromosomal DNA that originates from white blood cells. Generally, wbcDNA is gDNA and is assumed to be healthy DNA.

The term "tissue nucleic acid," "cancerous tissue DNA," or "tDNA" refers to nucleic acid including chromosomal DNA from tumor cells or other types of cancer cells that are obtained from cancerous tissue or a tumor. In some cases, tDNA is obtained from a biopsy of a tumor.

The term "alternative allele" or "ALT" refers to an allele having one or more mutations relative to a reference allele, e.g., corresponding to a known gene.

The term "sequencing depth" or "depth" refers to a total number of read segments from a sample obtained from an individual.

The term "alternate depth" or "AD" refers to a number of read segments in a sample that support an ALT, e.g., include mutations of the ALT.

The term "alternate frequency" or "AF" refers to the frequency of a given ALT. The AF may be determined by dividing the corresponding AD of a sample by the depth of the sample for the given ALT.

### II. EXAMPLE ASSAY PROTOCOL

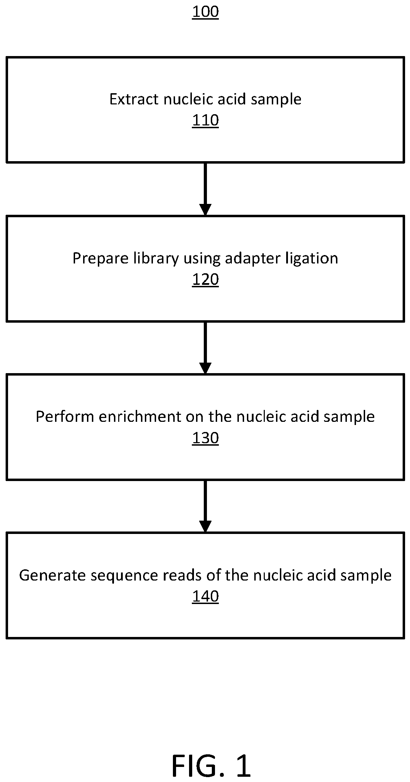

FIG. 1 is flowchart of a method 100 for preparing a nucleic acid sample for sequencing according to one aspect of the disclosure. The method 100 includes, but is not limited to, the following steps. For example, any step of the method 100 may comprise a quantitation substep for quality control or other laboratory assay procedures known to one skilled in the art.

In step 110, a nucleic acid sample (DNA or RNA) is extracted from a subject. In the present disclosure, DNA and RNA may be used interchangeably unless otherwise indicated. That is, the following embodiments for using error source information in variant calling and quality control may be applicable to both DNA and RNA types of nucleic acid sequences. However, the examples described herein may focus on DNA for purposes of clarity and explanation. The sample may be any subset of the human genome, including the whole genome. The sample may be extracted from a subject known to have or suspected of having cancer. The sample may include blood, plasma, serum, urine, fecal, saliva, other types of bodily fluids, or any combination thereof. In some cases, the sample can include tissue or bodily fluids extracted from tissue. In some embodiments, methods for drawing a blood sample (e.g., syringe or finger prick) may be less invasive than procedures for obtaining a tissue biopsy, which may require surgery. The extracted sample may include cfDNA and/or ctDNA. For healthy individuals, the human body may naturally clear out cfDNA and other cellular debris. If a subject has a cancer or disease, ctDNA in an extracted sample may be present at a detectable level for diagnosis.

Additionally, the extracted sample can include wbcDNA. Extracting 110 the nucleic acid sample can further include separating the cfDNA and/or ctDNA from the wbcDNA. Extracting the wbcDNA from the cfDNA and/or ctDNA can occur when the DNA is separated from the sample. In the case of a blood sample, the wbcDNA is obtained from a buff coat fraction of the blood sample. The wbcDNA can be sheared to obtain wbcDNA fragments less than 300 base pairs in length. Separating the wbcDNA from the cfDNA and/or ctDNA allows the wbcDNA to be sequenced independently from the cfDNA and/or ctDNA. Generally the sequencing process for wbcDNA is similar to the sequencing process for cfDNA and/or ctDNA.

In step 120, a sequencing library is prepared. During library preparation, unique molecular identifiers (UMI) are added to the nucleic acid molecules (e.g., DNA molecules) through adapter ligation. The UMIs are short nucleic acid sequences (e.g., 4-10 base pairs) that are added to ends of DNA fragments during adapter ligation. In some embodiments, UMIs are degenerate base pairs that serve as a unique tag that can be used to identify sequence reads originating from a specific DNA fragment. During PCR amplification following adapter ligation, the UMIs are replicated along with the attached DNA fragment, which provides a way to identify sequence reads that came from the same original fragment in downstream analysis.

In step 130, targeted DNA sequences are enriched from the library. During enrichment, hybridization probes (also referred to herein as "probes") are used to target, and pull down, nucleic acid fragments informative for the presence or absence of cancer (or disease), cancer status, or a cancer classification (e.g., cancer type or tissue of origin). For a given workflow, the probes may be designed to anneal (or hybridize) to a target (complementary) strand of DNA or RNA. The target strand may be the "positive" strand (e.g., the strand transcribed into mRNA, and subsequently translated into a protein) or the complementary "negative" strand. The probes may range in length from 10s, 100s, or 1000s of base pairs. In one embodiment, the probes are designed based on a gene panel to analyze particular mutations or target regions of the genome (e.g., of the human or another organism) that are suspected to correspond to certain cancers or other types of diseases. Moreover, the probes may cover overlapping portions of a target region. By using a targeted gene panel rather than sequencing all expressed genes of a genome, also known as "whole exome sequencing," the method 100 may be used to increase sequencing depth of the target regions, where depth refers to the count of the number of times a given target sequence within the sample has been sequenced. Increasing sequencing depth reduces required input amounts of the nucleic acid sample. After a hybridization step, the hybridized nucleic acid fragments are captured and may also be amplified using PCR.

In step 140, sequence reads are generated from the enriched DNA sequences. Sequencing data may be acquired from the enriched DNA sequences by known means in the art. For example, the method 100 may include next generation sequencing (NGS) techniques including synthesis technology (Illumina), pyrosequencing (454 Life Sciences), ion semiconductor technology (Ion Torrent sequencing), single-molecule real-time sequencing (Pacific Biosciences), sequencing by ligation (SOLiD sequencing), nanopore sequencing (Oxford Nanopore Technologies), or paired-end sequencing. In some embodiments, massively parallel sequencing is performed using sequencing-by-synthesis with reversible dye terminators.

In some embodiments, the sequence reads may be aligned to a reference genome using known methods in the art to determine alignment position information. The alignment position information may indicate a beginning position and an end position of a region in the reference genome that corresponds to a beginning nucleotide base and end nucleotide base of a given sequence read. Alignment position information may also include sequence read length, which can be determined from the beginning position and end position. A region in the reference genome may be associated with a gene or a segment of a gene. As cfDNA and/or ctDNA and wbcDNA are sequenced independently, sequence reads for both cfDNA and or ctDNA and wbcDNA are independently generated.

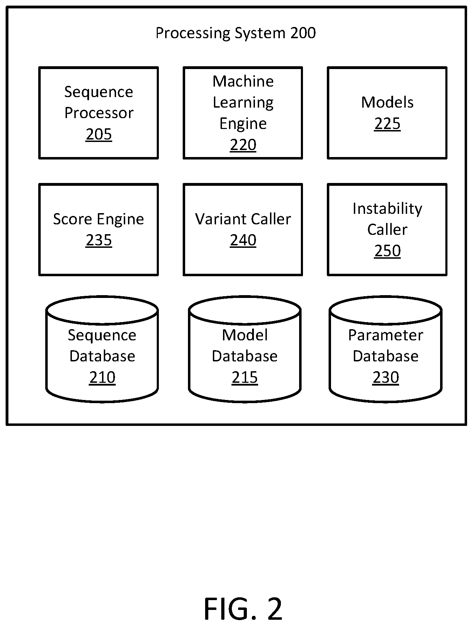

In various embodiments, a sequence read is comprised of a read pair denoted as *R*₁ and *R*₂. For example, the first read *R*₁ may be sequenced from a first end of a nucleic acid fragment whereas the second read *R*₂ may be sequenced from the second end of the nucleic acid fragment. Therefore, nucleotide base pairs of the first read *R*₁ and second read *R*₂ may be aligned consistently (e.g., in opposite orientations) with nucleotide bases of the reference genome. Alignment position information derived from the read pair *R*₁ and *R*₂ may include a beginning position in the reference genome that corresponds to an end of a first read (e.g., *R*₁) and an end position in the reference genome that corresponds to an end of a second read (e.g., *R*₂). In other words, the beginning position and end position in the reference genome represent the likely location within the reference genome to which the nucleic acid fragment corresponds. An output file having SAM (sequence alignment map) format or BAM (binary) format may be generated and output for further analysis such as variant calling, as described below with respect to FIG. 2.

### III. EXAMPLE PROCESSING SYSTEM

FIG. 2 is block diagram of a processing system 200 for processing sequence reads according to one aspect of the disclosure. The processing system 200 includes a sequence processor 205, sequence database 210, model database 215, machine learning engine 220, models 225 (for example, including one or more Bayesian hierarchical models or joint models), parameter database 230, score engine 235, variant caller 240, and instability caller 250.

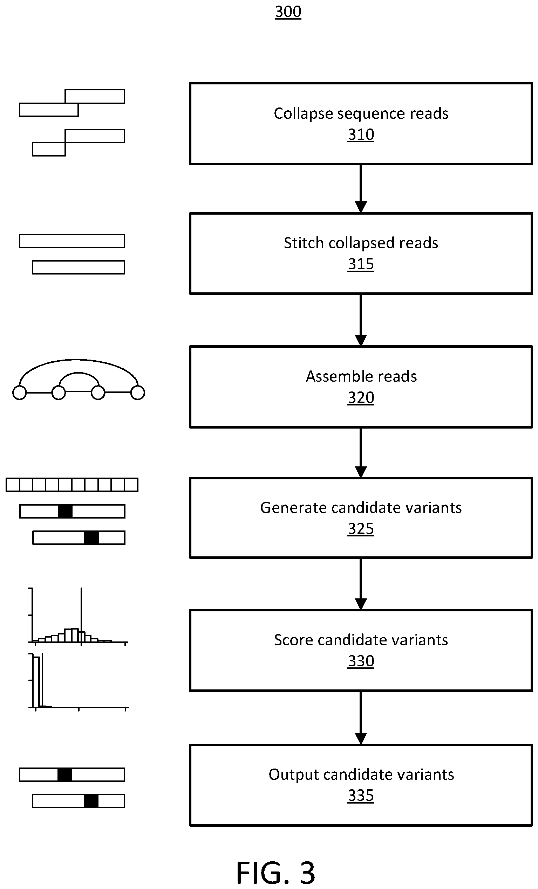

FIG. 3 is flowchart of a method 300 for determining variants of sequence reads according to one aspect of the disclosure. In some embodiments, the processing system 200 performs the method 300 to perform variant calling (e.g., for SNVs and/or indels) based on input sequencing data. Further, the processing system 200 may obtain the input sequencing data from an output file associated with nucleic acid sample prepared using the method 100 described above. The method 300 includes, but is not limited to, the following steps, which are described with respect to the components of the processing system 200. In other embodiments, one or more steps of the method 300 may be replaced by a step of a different process for generating variant calls, e.g., using Variant Call Format (VCF), such as HaplotypeCaller, VarScan, Strelka, or SomaticSniper.

At step 310, the sequence processor 205 collapses aligned sequence reads of the input sequencing data. In one embodiment, collapsing sequence reads includes using UMIs, and optionally alignment position information from sequencing data of an output file (e.g., from the method 100 shown in FIG. 1) to collapse multiple sequence reads into a consensus sequence for determining the most likely sequence of a nucleic acid fragment or a portion thereof. Since the UMIs are replicated with the ligated nucleic acid fragments through enrichment and PCR, the sequence processor 205 may determine that certain sequence reads originated from the same molecule in a nucleic acid sample. In some embodiments, sequence reads that have the same or similar alignment position information (e.g., beginning and end positions within a threshold offset) and include a common UMI are collapsed, and the sequence processor 205 generates a collapsed read (also referred to herein as a consensus read) to represent the nucleic acid fragment. The sequence processor 205 designates a consensus read as "duplex" if the corresponding pair of collapsed reads have a common UMI, which indicates that both positive and negative strands of the originating nucleic acid molecule is captured; otherwise, the collapsed read is designated "non-duplex." In some embodiments, the sequence processor 205 may perform other types of error correction on sequence reads as an alternate to, or in addition to, collapsing sequence reads.

At step 315, the sequence processor 205 stitches the collapsed reads based on the corresponding alignment position information. In some embodiments, the sequence processor 205 compares alignment position information between a first read and a second read to determine whether nucleotide base pairs of the first and second reads overlap in the reference genome. In one use case, responsive to determining that an overlap (e.g., of a given number of nucleotide bases) between the first and second reads is greater than a threshold length (e.g., threshold number of nucleotide bases), the sequence processor 205 designates the first and second reads as "stitched"; otherwise, the collapsed reads are designated "unstitched." In some embodiments, a first and second read are stitched if the overlap is greater than the threshold length and if the overlap is not a sliding overlap. For example, a sliding overlap may include a homopolymer run (e.g., a single repeating nucleotide base), a dinucleotide run (e.g., two-nucleotide base sequence), or a trinucleotide run (e.g., three-nucleotide base sequence), where the homopolymer run, dinucleotide run, or trinucleotide run has at least a threshold length of base pairs.

At step 320, the sequence processor 205 assembles reads into paths. In some embodiments, the sequence processor 205 assembles reads to generate a directed graph, for example, a de Bruijn graph, for a target region (e.g., a gene). Unidirectional edges of the directed graph represent sequences of k nucleotide bases (also referred to herein as "k-mers") in the target region, and the edges are connected by vertices (or nodes). The sequence processor 205 aligns collapsed reads to a directed graph such that any of the collapsed reads may be represented in order by a subset of the edges and corresponding vertices.

In some embodiments, the sequence processor 205 determines sets of parameters describing directed graphs and processes directed graphs. Additionally, the set of parameters may include a count of successfully aligned k-mers from collapsed reads to a k-mer represented by a node or edge in the directed graph. The sequence processor 205 stores, e.g., in the sequence database 210, directed graphs and corresponding sets of parameters, which may be retrieved to update graphs or generate new graphs. For instance, the sequence processor 205 may generate a compressed version of a directed graph (e.g., or modify an existing graph) based on the set of parameters. In one use case, in order to filter out data of a directed graph having lower levels of importance, the sequence processor 205 removes (e.g., "trims" or "prunes") nodes or edges having a count less than a threshold value, and maintains nodes or edges having counts greater than or equal to the threshold value.

At step 325, the variant caller 240 generates candidate variants from the paths assembled by the sequence processor 205. In one embodiment, the variant caller 240 generates the candidate variants by comparing a directed graph (which may have been compressed by pruning edges or nodes in step 310) to a reference sequence of a target region of a genome. The variant caller 240 may align edges of the directed graph to the reference sequence, and records the genomic positions of mismatched edges and mismatched nucleotide bases adjacent to the edges as the locations of candidate variants. Additionally, the variant caller 240 may generate candidate variants based on the sequencing depth of a target region. In particular, the variant caller 240 may be more confident in identifying variants in target regions that have greater sequencing depth, for example, because a greater number of sequence reads help to resolve (e.g., using redundancies) mismatches or other base pair variations between sequences.

In one embodiment, the variant caller 240 generate candidate variants using a model 225 to determine expected noise rates for sequence reads from a subject. The model 225 may be a Bayesian hierarchical model, though in some embodiments, the processing system 200 uses one or more different types of models. Moreover, a Bayesian hierarchical model may be one of many possible model architectures that may be used to generate candidate variants and which are related to each other in that they all model position-specific noise information in order to improve the sensitivity/specificity of variant calling. More specifically, the machine learning engine 220 trains the model 225 using samples from healthy individuals to model the expected noise rates per position of sequence reads.

Further, multiple different models may be stored in the model database 215 or retrieved for application post-training. For example, a first model is trained to model SNV noise rates and a second model is trained to model indel noise rates. Further, the score engine 235 may use parameters of the model 225 to determine a likelihood of one or more true positives in a sequence read. The score engine 235 may determine a quality score (e.g., on a logarithmic scale) based on the likelihood. For example, the quality score is a Phred quality score *Q* = -10·*log*₁₀ *P*, where P is the likelihood of an incorrect candidate variant call (e.g., a false positive).

At step 330, the score engine 235 scores the candidate variants based on the model 225 or corresponding likelihoods of true positives or quality scores. Training and application of the model 225 is described in more detail below.

At step 335, the processing system 200 outputs the candidate variants. In some embodiments, the processing system 200 outputs some or all of the determined candidate variants along with the corresponding scores. Downstream systems, e.g., external to the processing system 200 or other components of the processing system 200, may use the candidate variants and scores for various applications including, but not limited to, predicting presence of cancer, disease, or germline mutations.

Candidate variants are outputted for both cfDNA and/or ctDNA and wbcDNA. Herein, generally, candidate variants for wbcDNA are "normals" while candidate variants for cfDNA and/or ctDNA are "variants." Various detection methods and models can compare variants to normals to determine if the variants include signatures of cancer or any other disease. In various embodiments, normals and variants can be generated using any other process, any number of samples (e.g., a tumor biopsy or blood sample), or accessed from a database storing candidate variants.

### IV. DETERMINING MICROSATELLITE INSTABILITY IN A SAMPLE

Processing system 200 generates variants and normals that can be used to detect cancer in a subject, or predict a likelihood that a subject has cancer, from a cfDNA sample. Some cancers can include microsatellite instability (MSI). Microsatellite instability is the condition of genetic hypermutability that results from impaired DNA mismatch repair (MMR). The presence of MSI represents phenotypic evidence that MMR is not functioning normally and can, in some cases, be associated with various forms of cancer. Detecting MSI, or determining a likelihood of MSI, in a subject can be useful for a variety of reasons: informing a method of treatment for the subject, determining the likelihood of curing the subject, determining the severity of the cancer, determining the progression of the cancer, etc.

Processing system 200 includes an instability caller 250 configured to determine a likelihood that generated variants include MSI. Instability caller 250 inputs variants and normals (i.e., test reads and control reads) for a sample and determines a likelihood that the sample includes MSI based on an analysis and comparison of the inputted sequences. While instability caller 250 detects MSI in variants generated by processing system 200, instability caller 250 can be used to detect MSI in any other set of DNA sequence reads.

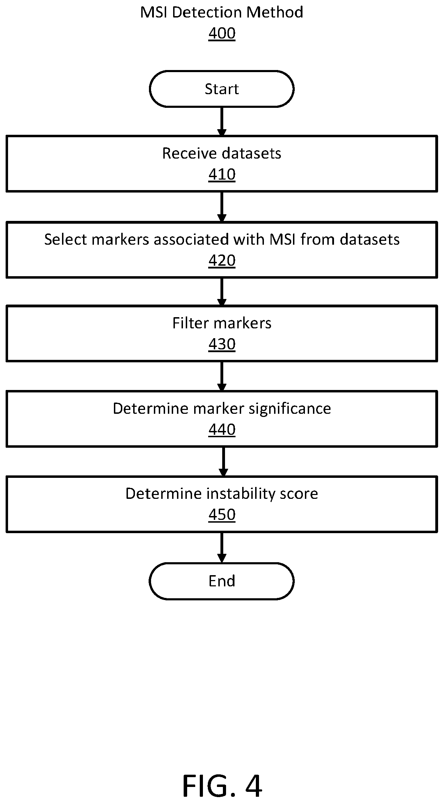

FIG. 4 illustrates a flow diagram illustrating a MSI detection method 400 performed by instability caller 250 of processing system 200, in one example embodiment. In this example, MSI detection method 400 includes, but is not limited to, the following steps.

At step 410, sequencing data is obtained from a sample (e.g., using method 300, or accessing stored sequencing data). Generally, the data obtained from a sample includes a number of sequencing reads from cfDNA and wbcDNA obtained from a blood sample of a subject. Sequencing reads (i.e., reads) from cfDNA can include both DNA associated with cancer (i.e., ctDNA) and DNA not associated with cancer. In some samples, cfDNA can include gDNA from white blood cell fragments.

Variant caller 240 can call individual positions within the ctDNA as variants (i.e., test reads) using processing system 200. Reads from gDNA are obtained from wbcDNA or white blood cell fragments in the sample, and are, in this case, not associated with cancer. Variant caller 240 can call individual positions within the gDNA as normals (i.e., control reads) using processing system 200. As previously described, test reads and control reads (i.e., sample reads, in aggregate) each have various characteristics such as read depth, allele frequency, bag depth, etc. Processing system 200 can analyze the differences between the test reads, the control reads, and their respective characteristics to detect MSI in the sample.

At step 420, markers associated with MSI are selected from the received datasets. As referenced herein, a marker is one or more reference locations identified from a sequence read or a plurality of sequence reads at a given location of a reference genome. Markers are associated with nucleotides that can indicate cancer when MSI is detected. For any given marker, the microsatellite lengths, or microsatellite length distribution, can be similar between test reads and control reads. In samples that do not include MSI, microsatellite lengths (or length distributions) are similar between test reads and control reads. Conversely, in samples that include MSI indicative of cancer, microsatellite lengths (or lengths distributions) are variable in test reads when compared to control reads.

Each marker can be given a marker identifier (i.e., Marker ID) describing characteristics of the marker. For example, a Marker ID can be "BAM chr2 220439700 CTCTG 5[CT] GCTGA HOM," where BAM is the read source, chr2 indicates the marker is on the second chromosome, 220439700 is a site identifier, CTCTG 5[CT] GCTGA are the set of nucleotides included in the marker, CTCTG and GCTGA are the flanking sequences of the marker, [CT] is a microsatellite in the marker (i.e., a subset of nucleotides that repeat), 5 is the microsatellite length (i.e., the number of times the microsatellite repeats) in the marker (e.g., 5 repeats of CT), and HOM is an indication of the zygosity of the marker (e.g., homozygous "HOM" or heterozygous "HET"). For each marker, any sample read that includes the marker is associated with that marker. Thus, each marker in a sample can have a number of test reads and control reads including that marker associated with it (i.e., marker reads).

At step 430, sample reads are filtered such that the specificity of the MSI detection method 400 increases. According to the invention, MSI detection method 400 removes heterozygous reads from sample reads such that determining marker significance and calculating instability scores uses only homozygous reads. In another example, detection method 400 removes marker reads if the marker reads (test, control, or both) do not have a sufficient read depth. In various other configurations the MSI detection algorithm filters the reads based on any of bag size, bag depth, marker location, microsatellite nucleotides, or any other characteristic of a read that can be filtered.

At step 440, MSI detection method 400 determines a marker significance for each marker. Marker significance represents the significance and viability for the marker in detecting MSI. Here, the viability of a marker describes a level of similarity between characteristics of test reads and control reads for the marker. The significance of a marker represents the statistical significance of test reads with dissimilar microsatellite lengths (e.g., tests reads include 5[CT], 6[CT], 7[CT], etc.) relative to the microsatellite length in the control reads (e.g., control reads include predominantly 5[CT]). Determining 440 marker significance can include calculating any number of marker scores (e.g., significance score, divergence score, entropy score, viability score, etc.) based on the sample reads as described in the Section titled "Determining Marker Significance." Each marker is determined to be significant or insignificant, with significant markers indicating that MSI is likely in that marker and insignificant markers indicating the MSI is unlikely in that marker.

At step 450, detection method 400 determines an instability score for the sample representing the likelihood that the sample contains MSI. In one example, the instability score is a ratio of significant to insignificant markers for the sample. In another example, a weighting function using determined marker significances and/or marker scores can be used to determine an instability score. If the instability score is above a threshold level instability the caller 250 calls the sample as including MSI.

The MSI detection method (400) of the invention detects a likelihood of cancer in an individual. The MSI detection method (400) may also detect the presence of cancer, a particular type of cancer, or a degree of cancer based on the instability score. In some cases, the MSI detection method informs a method of treatment based on the instability score.

### IV. A EXAMPLE OF MSI DETECTION IN A SAMPLE

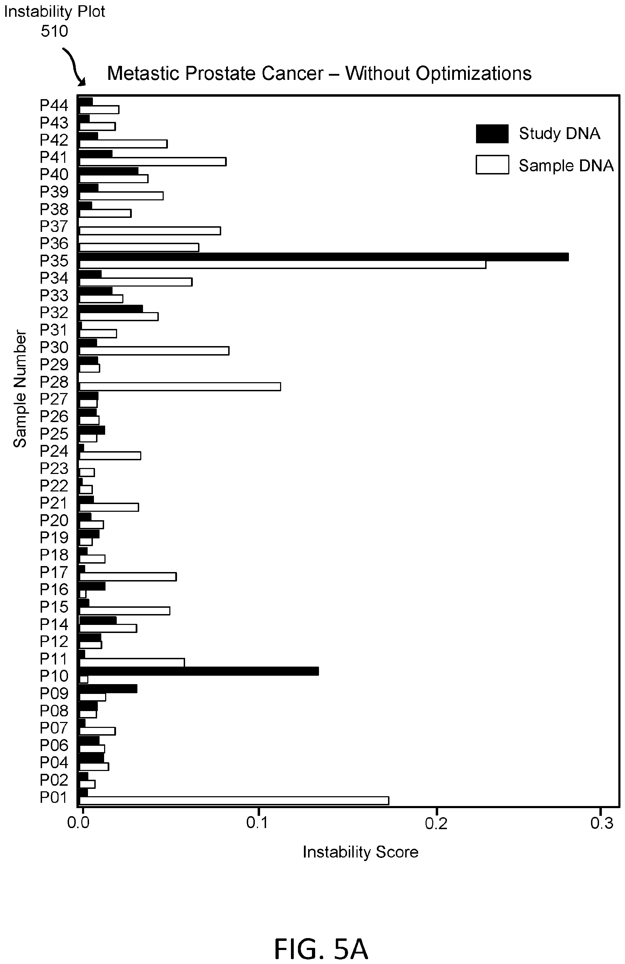

FIGS. 5A-5B are example instability plots comparing MSI detection methods for metastatic prostate cancer samples, according to one example embodiment. In instability plots 510 and 520, the y-axis indicates a sample number, with each sample number being a sample from a different subject. The x-axis is the instability score representing a likelihood that the sample includes MSI. The instability score for Study DNA samples are shown with darker bars and the instability score for Sample DNA samples are shown with lighter bars. Study DNA samples are obtained from a prior art study that includes biopsies of tumors. Here, Study DNA includes both tDNA and cfDNA sequence reads. In Study DNA, tDNA and ctDNA (from the cfDNA) test reads are compared against gDNA control reads to determine the instability score for a given sample. Sample DNA is, generally, cfDNA sequence reads and can be obtained from a blood sample, extracted from a biopsy, or accessed from a sequence datastore (e.g., sequence database 210). In Sample DNA, ctDNA (from the cfDNA) tests reads are compared against gDNA (e.g., wbcDNA) test reads to determine the instability score for a sample.

FIG. 5A is an instability plot 510 showing the determined instability score of Study DNA samples and Sample DNA samples using a prior art method, according to one example embodiment. The prior art method provides more accurate detection of MSI for Study DNA than Sample DNA. Increased accuracy in MSI detection for Study DNA can be due to several contributing factors: a higher concentration of reads indicating MSI in tDNA compared to cfDNA; assumptions and errors included in the prior art method that generate MSI detection biases in ctDNA samples; and, the read depth for the Study DNA may be cause biases in MSI detection.

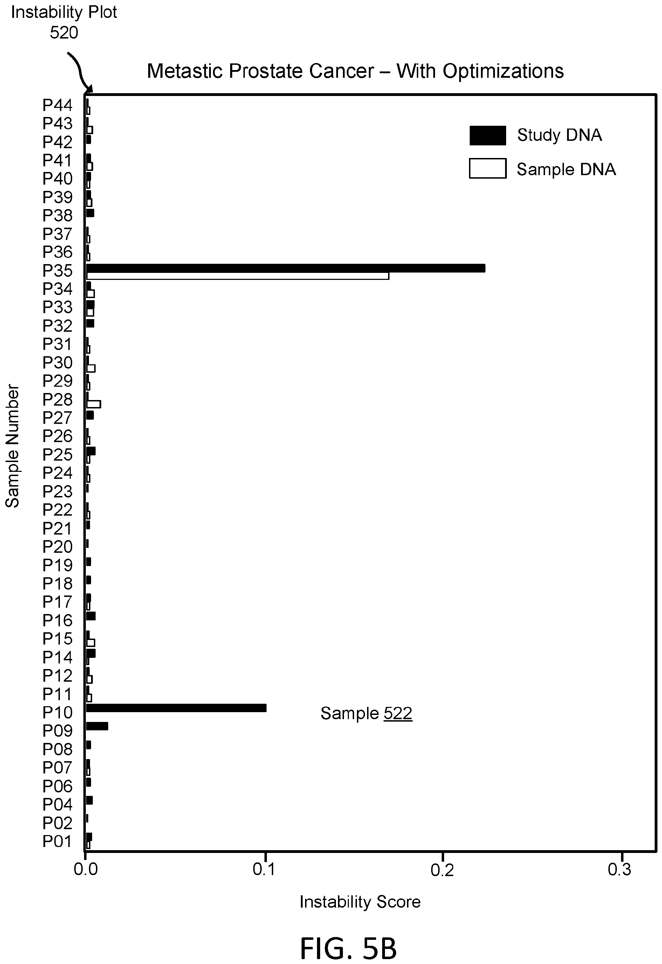

FIG. 5B is an instability plot 520 showing the determined instability score of Study DNA samples and Sample DNA samples using MSI detection method 400. Relative to prior art methods, MSI detection method 400 removes assumptions and errors that generate MSI detection biases, is configured for modern read depths, and is configured for detecting MSI in ctDNA samples. As a result, MSI detection method 400 determines instability scores largely similar between the Study DNA and Sample DNA. MSI detection method 400 also reduces the noise floor of the instability score for all samples.

In some cases, MSI detection using MSI detection method 400 in tDNA can be more accurate than in cfDNA. This can be caused by a higher concentration of reads indicating MSI in tDNA compared to cfDNA. For example, sample 522 of FIG. 5B indicates a high MSI likelihood in Study DNA (i.e., tDNA and cfDNA) but not Sample DNA (i.e., cfDNA). In this particular sample, there is not sufficient ctDNA in the Sample DNA to accurately determine the presence of MSI, but there is sufficient tDNA and ctDNA in the Study DNA to accurately determine the presence of MSI.

Herein, detection method 400 compares data from ctDNA test reads to gDNA control reads to detect MSI. However, various aspects of method 400 can be applied to detect MSI in any other type or set of sequence reads as long as the sequence reads include a set reads in which presence of MSI is unknown, and a set of reads in which MSI is known not to be present.

### V. DETERMINING MARKER SIGNIFICANCE

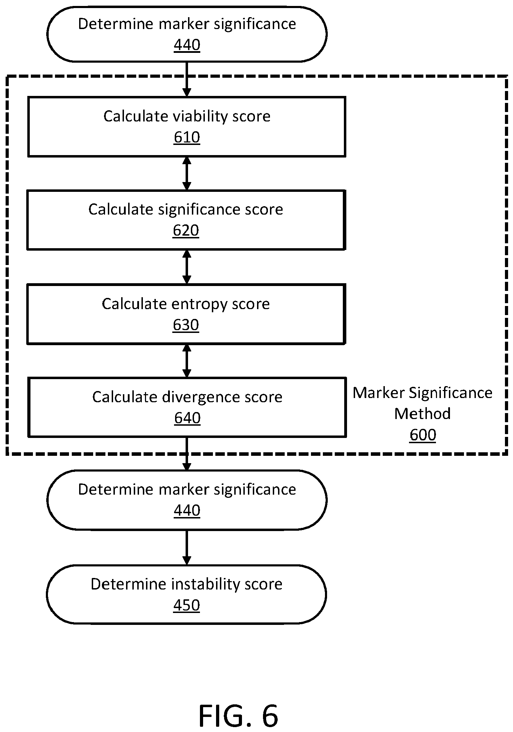

FIG. 6 illustrates a flow diagram illustrating a marker significance method 600 (i.e., significance method) to determine marker significance (e.g., step 440 of FIG. 4) performed in accordance to the detection method 400, in one example embodiment. In the illustrated example, method 600 includes, but is not limited to, the following steps, and the steps can be performed in any order, not performed, or performed multiple times.

Significance method 600 involves, in part, determining 440 marker significances. This includes calculating one or more of a variety of scores. Method 600 can calculate, for each marker, one or more of a viability score 610, a significance score 620, an entropy score 630, and a divergence score 640. MSI detection method 400 determines 440 a marker significance for each marker based on whichever scores are determined. The calculated scores provide quantitative methods for measuring the significance and viability of observed microsatellite length variations when determining marker significance. MSI detection method 400 determines 450 an instability score using the determined 440 marker significances.

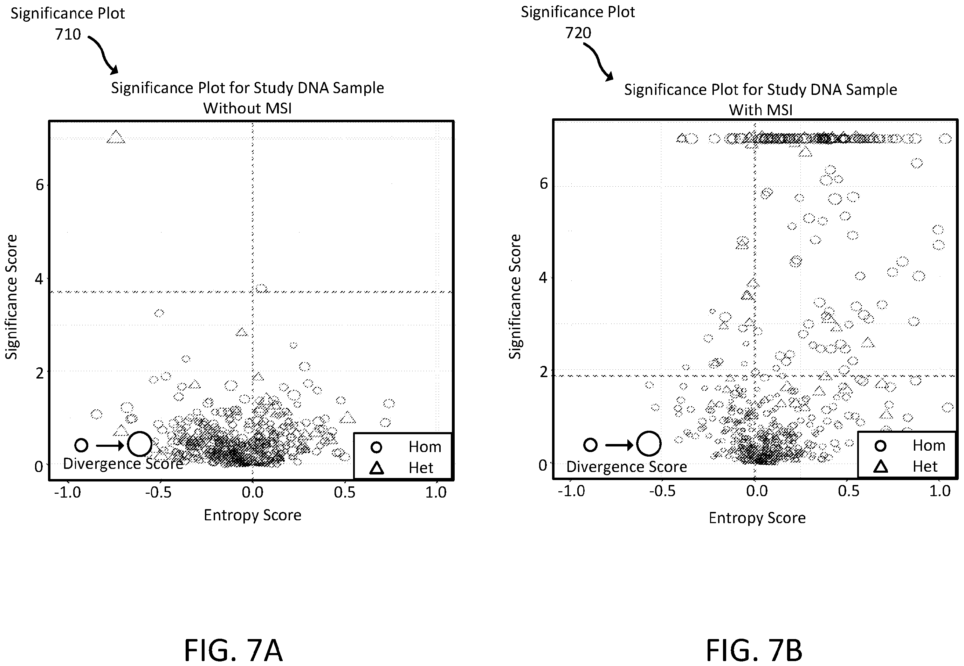

FIGS. 7A-7D are significance plots giving a visual example for determining marker significance from the calculated scores of method 400 for a set of markers (e.g., data points). In these figures, each marker within a sample is represented by a different data point in the plot. The data represented by each data point in the plot (e.g., the entropy score, the significance score, the divergence score, and the viability score) is used to determine marker significances for a sample. The determined marker significances, in aggregate, can be used to determine an instability score.

In FIGS. 7A-7D, the x-axis is the entropy score, the y-axis is the significance score, the shape of the data points represent the zygosity of the marker, specifically triangle data points are heterozygous markers and circular data points are homozygous markers. The relative size of the data points represent the divergence score (i.e., a small triangle represents a low divergence score and a large triangle represents a high divergence score, etc.). Further, only markers with a viability score above a threshold viability are illustrated on the significance plot (e.g., with a read depth above 20 reads). Calculation of the viability score, the significance score, the entropy score, and the divergence score are described in Sections V.A-V.D titled "Viability Score," "Significance Score," "Entropy Score," and "Divergence Score," respectively.

Significant markers, in general, have greater magnitude, positive-valued entropy, divergence, significance, and viability scores relative to insignificant markers. Visually, significant markers are illustrated as large data points near the top right of a significance plot. However, significant markers can be in other locations of the plot in various embodiments of method 600. Significance plots including a substantial portion of significant markers relative to insignificant markers are indicative of MSI in the sample. Visually, a sample including MSI includes a large portion of large data-points near the top-right of a significance plot. A sample not including MSI includes small data-points localized about the origin of the significance plot.

FIG. 7A-B are significance plots illustrating the scores used to determine marker significance for a pair of Study DNA samples. That is, test reads and control reads used to calculate the scores and determine marker significance in this sample include both gDNA and cfDNA. FIG. 7A is significance plot 710 for a sample showing that the calculated entropy score, significance score, and divergence score are low values for a large portion of the markers, according to one example embodiment. Many of the markers are determined 440 to be insignificant markers based on the scores. Accordingly, significance plot 710 indicates the sample does not include MSI and that the instability score is low. Conversely, FIG. 7B is a significance plot 720 for a different sample where the entropy score, significance score, and divergence score are high for a large portion of the markers, according to one example embodiment. Many of the markers are determined to be significant based on the scores. Consequently, significance plot 720 indicates that the sample includes MSI and the instability score is high.

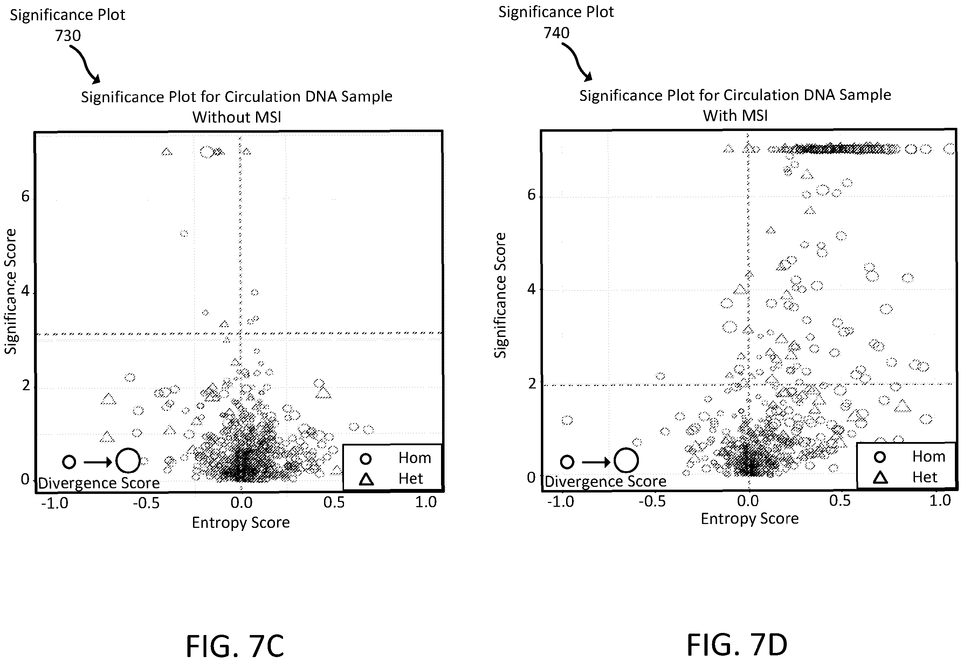

FIG. 7C-7D are significance plots used to determine marker significance in a cell-free DNA sample. FIG. 7C is a significance plot 730 showing a large number of insignificant markers indicating the sample does not include MSI, according to one example embodiment. FIG 7D is a significance plot 740 showing a large number of significant markers indicating the sample includes MSI, according to one example embodiment.

As discussed above, each data point in a significance plot (e.g., in FIGs. 7A-7D) is a representation of scores calculated based on test reads and control reads associated with a marker in a sample. The underlying data used to calculate these scores for a single data point / marker in a significance plot can be illustrated individually in a marker plot.

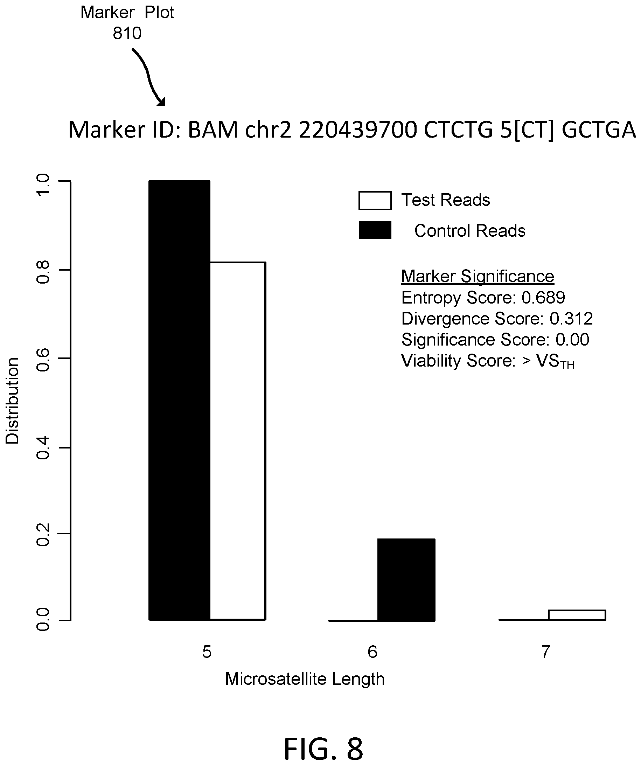

For example, FIG. 8 illustrates a marker plot 810 illustrating the distribution of the microsatellite lengths (i.e., number of repeats for a nucleotide set) for a marker in circulation DNA, according to one embodiment. The x-axis is the observed microsatellite length of a read, the y-axis is the (empirically determined) distribution of microsatellite lengths based on the reads obtained for the sample, the red bars are microsatellite lengths observed for test reads (e.g., ctDNA), and the black bars are microsatellite lengths observed for control reads (i.e., gDNA).

Marker plot 810 includes the marker identifier. In marker plot 810, the marker identifier indicates that the marker reads originate from a BAM file, the nucleotides are on chromosome two, has location identifier 220439700, and includes the set of nucleotides CTCTG 5[CT] GCTGA. For this marker, the microsatellite is the CT nucleotides with an expected microsatellite length of 5 (e.g., 5[CT]) in a sample without MSI instability. In this example, the microsatellite length is 5 in nearly all of the control reads. However, there is variation in the microsatellite length in the test reads with approximately 80 % of test reads having length 5, approximately 18% of test reads length having 6 repeats, and <2% of test reads having length 7. The illustrated variation of microsatellite length in marker plot 810 between test reads and control reads can, in some cases, be indicative of MSI instability in the sample.

The marker illustrated in marker plot 810 has a calculated entropy score of 0.689, divergence score of 0.312, significance score of 0.000. The calculated viability score is above a threshold viability score (i.e., VS_{TH}). Based on the scores, the marker in marker plot 810 determined to be significant and may indicate MSI instability.

### V.A VIABILITY SCORE

Returning to the method of FIG. 6, at step 610, significance method 600 calculates a viability score representing a level of similarity between characteristics of test reads and control reads for each marker. Significance method 600 determines a high viability score for a marker if characteristics of the test reads and control reads are similar and a low viability score for a marker if characteristics of the test reads and control reads are dissimilar.

As described above, characteristics of test reads and control reads can be dissimilar for any number of reasons. In one example, MSI detection method 400 can apply an error correction model to sample reads used for determining marker significance and instability score. The error correction model can determine, and sometimes correct, sample reads that include measurement errors.

In one example, correcting sample reads that include measurement errors can include removing reads from MSI detection method 400, but can also include other correction methods. In various configurations, the error correction model can include correction for one or more of unique molecular index correction, duplex correction, stitching, or positional error correction, and other error correction techniques. The error correction model can be more efficient for correcting some types of sample reads (and any systematic process errors used to produce those specific types of reads) over others. Here, for example, the error correction model is more efficient in correcting errors in test reads rather than control reads. However, in various other configurations, the error correction model can be more efficient in correcting any subset of system reads rather than another subset of system reads (e.g., reads for a certain site, reads for a certain marker, etc.). Correcting errors using the error correction model can change the characteristics of sample reads such that the test reads characteristics change relative to the control reads. The changed characteristics of the test reads affect the viability score of the marker. In similar examples, characteristics of control reads can change relative to test reads, control reads can change similarly to test reads, etc. Examples of sample processing characteristics that can change due to error correction can include any of bag size, length distribution, duplex rate, or sequence depth, etc.

Significance method 600 measures characteristics of test reads and control reads after applying the error correction model and calculates a viability score. In various other examples, significance method 600 can determine a viability score between any two sets of sequence reads for a given marker. Here, the viability score is a quantification of how similar the characteristics of the test reads and control reads are after error correction. For example, using the example that the error correction is more efficient at correcting test reads rather than control reads, the error correction model detects measurement errors in 30% of test reads 10% of control reads for a given marker. In this example, the error correction model corrects the errors by removing the reads including measurement errors, and, as such, the read depth for the test reads is much lower than the read depth for the control reads (assuming a similar un-corrected read depth pre-correction). Thus, significance method 600 determines a low viability score for the marker based on the discrepancy in read depths between test reads and control reads. In a counter-example, significance method 600 determines a high viability score for markers with highly similar characteristics (e.g., similar bag size).

The preceding examples of calculating a viability score are given for ease of understanding. However, the viability score can be calculated based on any number of characteristics and can quantify differences between characteristics in any number of ways (e.g., weighting, ratio analysis, etc.). For example, in an embodiment, the viability score can only be high if the number of test reads and control reads is the same. Alternatively, if there is a difference between the number of test reads and the number of control reads for a marker, the viability score for that marker will be low.

The calculated viability score can be used to determine 440 marker significance for each marker. In one example, when the viability score for a marker is below a threshold viability score, the marker is not used to calculate the instability score for the sample. That is, the marker is neither significant nor insignificant and removed from instability score calculations. Alternatively, the marker can be determined to be insignificant if the viability score is below a threshold.

Similarly, the calculated viability score can be used to determine 450 the instability score of the sample. For example, markers with very highly similar characteristics can have a viability score greater than markers with slightly similar characteristics. In this case, MSI detection method 400 may weight marker significances higher for markers with a higher viability score than markers with lower viability scores when determining the instability score for the sample.

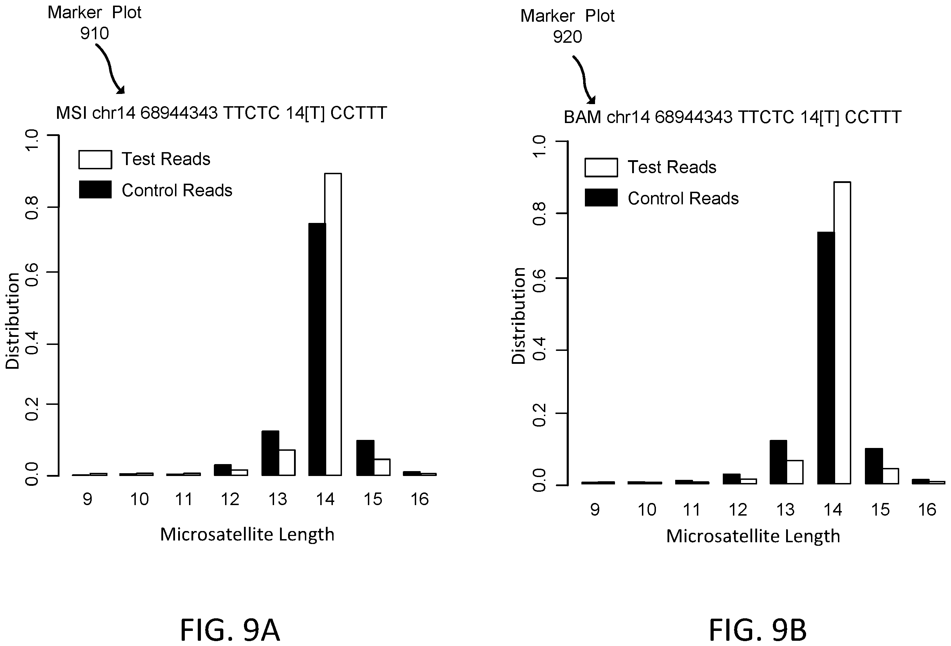

FIG. 9A-9D illustrate the effects of applying an error correction model on sample reads on a marker of a circulation DNA sample resulting in a high viability score.

FIG. 9A-9B are marker plots for a marker after error correction, the plots comparing a prior art method and the MSI detection method 400 for a sample including circulation DNA. In FIG. 9A, marker plot 910 illustrates the distribution of microsatellite lengths using the prior art method (without error correction, or minimal error correction), according to one example embodiment. In FIG. 9B, marker plot 920 illustrates the distribution of microsatellite lengths using MSI detection method 400 after applying an error correction model, according to one example embodiment. Here, both marker plot 910 and marker plot 920 are largely similar indicating that the error correction model did not affect the distribution of microsatellite length for this particular marker in the sample.

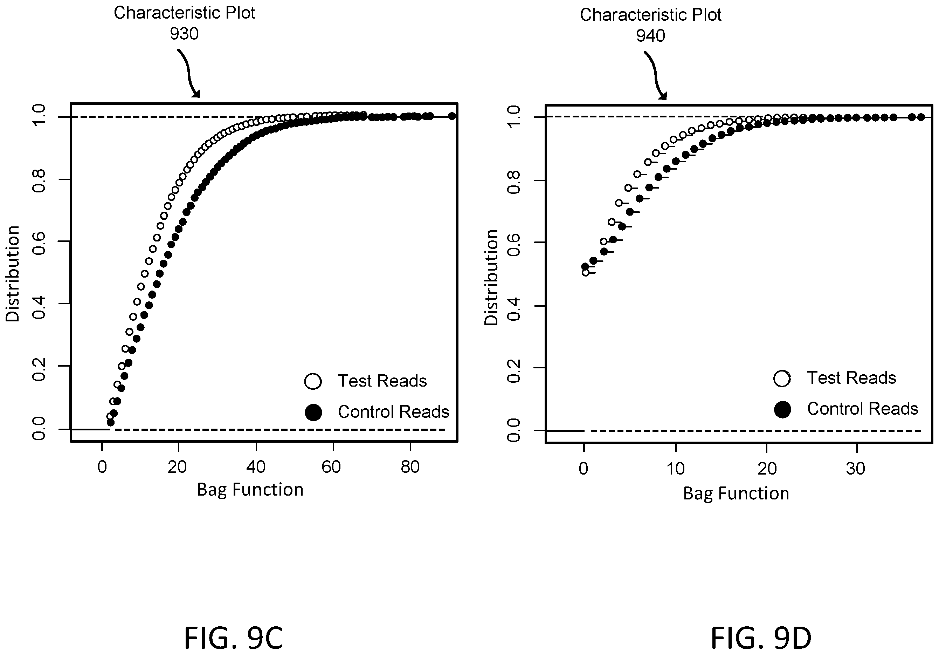

FIG. 9C-9D are characteristic plots illustrating a measure of similarity for a characteristic of sample reads for the marker of FIG. 9B, according to one example embodiment. The x-axis is a function of the number of reads in each bag (i.e., a particular UMI/position combination used during error correction). The y-axis is the distribution of the x-axis function. The red data points indicate the distribution of the function for test reads and the black points indicate the distribution of the function for control reads.

FIG. 9C is a characteristic plot 930 illustrating the similarity between the test reads and control reads for a first characteristic (i.e., bag size sum) of the sample reads, according to one example embodiment. The function of the x-axis in characteristic plot is the bag size sum. The bag size sum is the sum of the forward and reverse reads in the bag for the marker. A data point on characteristic plot 930 shows that for a given bag having a number of forward and reverse reads (x), a fraction (y) of bags have the same sum of forward and reverse reads in the bag. Here, the bag size sum is largely similar for both the test reads and the control reads. Thus, the MSI detection algorithm calculates that the viability score is high based on this characteristic of the sample reads.

FIG. 9D is a characteristic plot 940 illustrating the similarity between the test reads and control reads for a second characteristic (i.e., bag size min) of the reads, according to one example embodiment. The function of the x-axis in characteristic plot 940 is the bag size min. The bag size min is the minimum count of the forward and reverse reads in the bag for the marker. In characteristic plot 940, every bag of size 0 indicates that the bag included a single strand of DNA (i.e., was not duplex). A data point on characteristic plot 940 shows that for a given bag having a minimum count of forward and reverse reads (x), a certain fraction (y) of the bags have that minimum number of forward and reverse reads in the bag. In characteristic plot 940, bag size min distribution is largely similar for both the test reads and control reads. Thus, MSI detection method 400 calculates that the viability score is high based on this characteristic. FIGS. 9C-9D suggest that the error correction model is similarly efficient for cell-free variant DNA and white blood cell DNA for this marker.

As some of the bags were not duplex, the data points do not show a full distribution across the y-axis. The lack of full distribution in characteristic plot 940 indicates that approximately 50% of the test reads were not duplex, and that approximately 50% of the control reads were not duplex.

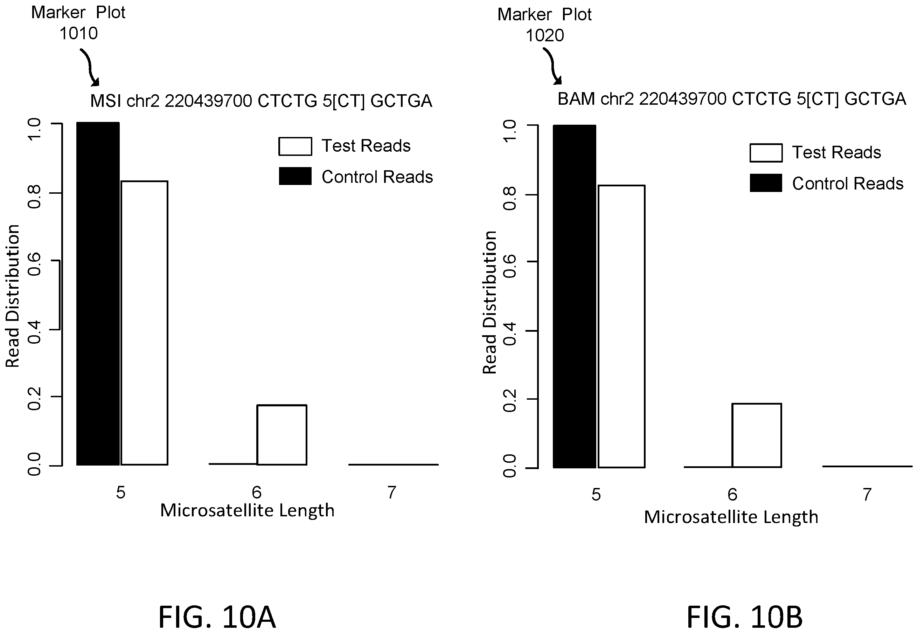

FIG. 10A-10D illustrate the effects of applying an error correction model on sample reads of a marker for a circulation DNA sample resulting in a low viability score.

FIG. 10A-10B are marker plots for a marker after error correction, the plots comparing a prior art method and the MSI detection method 400, respectively. FIG. 10A is a marker plot 1010 illustrating the variation in microsatellite length for a marker using a prior art method (with minimal, or no, error correction), according to one example embodiment. FIG. 10B is a marker plot 1020 illustrating the variation in microsatellite length for the same marker after applying an error correction model using MSI detection method 400, according to one example embodiment. Here, both marker plot 1010 and 1020 are largely similar indicating that the error correction model did not affect the distribution of microsatellite repeat lengths in the sample.

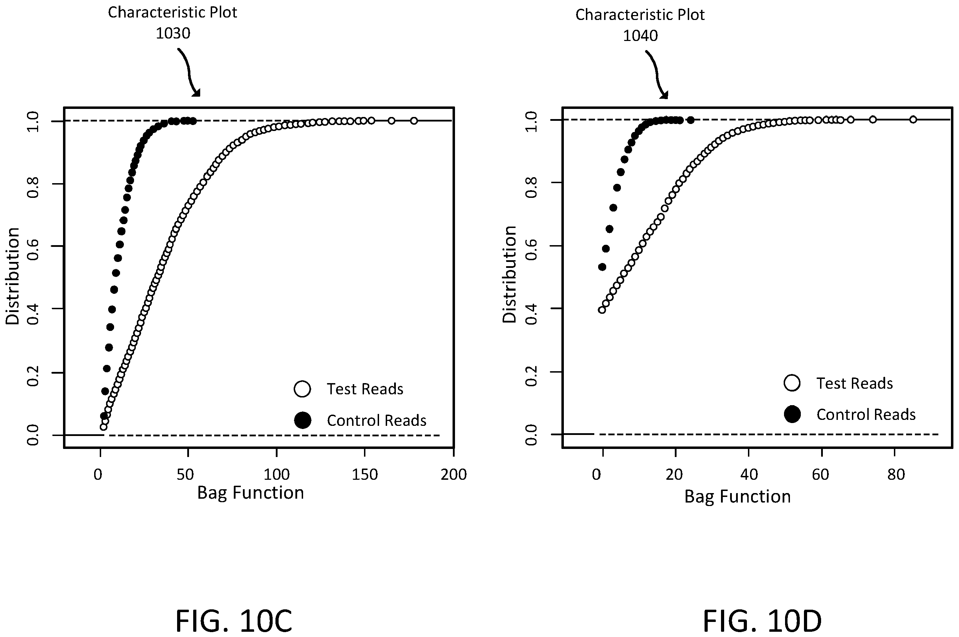

FIG. 10C-10D are characteristic plots illustrating a measure of similarity for a characteristic of test reads and control reads for the marker of FIG. 10B, according to one example embodiment. FIGs. 10C and 10D are similar to FIGs. 9C-9D. That is, FIG. 10C is a characteristic plot 1030 illustrating a measure of similarity for the bag size sum of the marker between test reads and control reads after error correction, according to one example embodiment. FIG. 10D is a characteristic plot 1040 illustrating a measure of similarity for the bag size min of the marker between test reads and control reads after error correction, according to one example embodiment. In the examples of FIG. 10C-10D the observed characteristics (i.e., bag size sum and bag size min) are largely dissimilar for test reads and control reads. The differential between the distributions (i.e., a greater distribution of test reads relative to control reads) indicates that the error correction model was more efficient for the test reads rather than the control reads. Thus, MSI detection method 400 calculates that the viability score is low based on these characteristics. In some configurations, if the calculated viability score is below a threshold viability score, the markers are not included in determining marker significance or determining an instability score.

As some of the bags were not duplex, the data points do not show a full distribution across the y-axis. The lack of full distribution in characteristic plot 940 indicates that approximately 60% of the test reads were not duplex, and that approximately 40% of the control reads were not duplex. In some configurations, this difference can generate a low viability score for the marker.

### V.B SIGNIFICANCE SCORE

Returning to FIG. 6, at step 620, method 600 calculates a significance score for each marker representing the statistical significance of test reads with a variation in a microsatellite length of a microsatellite relative to microsatellite length of the same microsatellite in the control reads. In particular, the significance score quantifies the statistical significance of the variation in the microsatellite length distribution of test reads relative to control reads.

In one example embodiment, the significance score for a marker is the p-value of a Chi-Squared test comparing test reads to control reads. In calculating the Chi-squared test, the observed microsatellite lengths for the test reads and the expected value is the average microsatellite length of the control reads. Calculating the Chi-squared test produces a Chi-squared value. The p-value of the Chi-squared test is calculated with the Chi-squared value and 2 degrees of freedom. The calculated p-value is the significance score. In other configurations, the significance score can be calculated using other methods of quantifying a statistical significance for a marker.

A significance test is applied to the calculated significance scores to determine if a marker is significant. In one example embodiment, the significance test compares the significance score to a significance threshold α (i.e., α = .01, α =.05, α = 0.10, etc.) to determine if the marker is significant. In the example where the significance score is a p-value of the chi-squared test, markers are significant if the p-values are below the significance threshold α. In another example embodiment, the significance test applies a methodology to reduce the rate of false positives in marker significance. For example, the significance test can apply the Benjamani-Hochberg correction procedure using a false discovery rate β (e.g., 5%). In this example, markers that pass the Benjamani-Hochberg correction procedure are significant markers. In various other example embodiments, the MSI detection method can use any number or types of significance tests to determine marker significance.

### V.C ENTROPY SCORE

At step 630, significance method 600 calculates an entropy score for each marker representing the entropy of each marker. Here, the entropy is the entropy calculated in information theory. That is, the entropy is the average amount of information produced by a stochastic set of data. In the context of detection method 400, the entropy score is a measure of a difference in entropy of the marker between the test reads and the control reads.

To calculate the entropy score for a marker, the significance method 600 calculates an entropy metric for the test reads and control reads separately. Entropy for each marker is associated with the negative logarithm of the probability mass function for the set of nucleotides in the marker. Calculating the entropy metric for the test reads and control reads for a marker includes determining an entropy value for each read. The entropy value for a test read (or control read) is the probability of observing a microsatellite length in the test read (or control read) times the logarithm of the probability of observing the microsatellite length of the test read (or control read). The entropy metric for the test reads and control reads in the marker is the summation of the entropy values for the test reads and control reads, respectively.

In a sample including MSI, there is a variation across reads in microsatellite length which generates a high entropy metric. In a sample without MSI, there is much less variation in microsatellite lengths reads which generates a low entropy metric. Thus, for a set of test or control reads for a marker, a high entropy metric can be indicative of MSI.

Significance method 600 calculates the entropy score for a marker by comparing the entropy metric of the test reads to the entropy metric of control reads. Generally, the entropy score is the entropy metric of the test reads less the entropy metric of the control reads. Significance method 600 compares the entropy values between test reads and control reads because some control reads can have microsatellite length variation while not being indicative of MSI associated with cancer. When the entropy metric for the test reads is significantly greater than the entropy metric of the control reads the difference indicates that the microsatellite length variation can be indicative of MSI associated with cancer. Thus, the entropy score is high when a marker can be indicative of MSI, and low when the marker is not.

The MSI detection method 400 evaluates the entropy score for each marker to determine marker significance. In one example embodiment, a marker is significant if the entropy scores indicates that the test read is more disordered than the control read. In another example embodiment, the entropy score is compared to a threshold entropy score for each marker, and if the entropy score is greater than the threshold entropy score the marker is a significant marker.

### V.D DIVERGENCE SCORE

Returning to FIG. 6, at step 640, significance method 600 calculates a divergence score for each marker representing a measure of the relative entropy between test reads and control reads for the marker. The divergence score measures how the probability distribution of the microsatellite lengths for the test reads diverge from the probability distribution of microsatellite length for the control reads.

In one example embodiment, the divergence score is the Jensen-Shannon divergence of test reads and control reads for each marker. In this case, to calculate the divergence score, significance method 600 determines a probability for measuring each observed microsatellite length for test reads and control reads, respectively. For every observed microsatellite length, significance method 600 calculates a first length probability value and a second length probability value. The first length probability value compares the probability of observing the microsatellite length in test reads to a logarithmic ratio of the probability for observing the microsatellite lengths in test reads to the probability of observing the microsatellite lengths in an average of test reads and control reads. The second length probability value compares the probability of observing the microsatellite length in control reads to a logarithmic ratio of the probability for observing the microsatellite lengths in control reads to the probability of observing the microsatellite lengths in an average of test reads and control reads. The divergence score is the sum of the first and second length values for all observed microsatellite lengths. Alternatively stated, the divergence score is the expectation of observing a microsatellite length given the distributions of the test reads and control reads. In various other embodiments, the divergence score can be any scoring methodology that compares the probability distributions of test reads and control reads.

In the context of significance method 600, the divergence score measures relative differences between the probability distributions for observing a set of microsatellite lengths. Thus, in a sample that includes variations in microsatellite lengths in control reads dissimilar to variations in microsatellite lengths in test reads (or vice-versa), the divergence score is high. In sample that does not include variations in microsatellite lengths between test reads and control reads, or includes similar variations in microsatellite lengths between test reads and control reads, the divergence score is low. Generally, MSI causes larger variations in microsatellite lengths in test reads relative to control reads and, hence, the divergence score for a sample including MSI is high.

The significance method 600 evaluates the divergence score for each marker to determine 440 marker significance. In one example embodiment, the divergence score is compared to a threshold divergence score for each marker. The significance method 600 defines a marker as significant if the divergence score is greater than the threshold divergence score.

### VI. FILTERING

As previously described, the MSI detection method can filter the test reads based on any number of criteria.

### VI.A ZYGOSITY FILTERING

In heterozygous reads, copy number aberrations can generate biases in calculating scores used for determining marker significance and instability scores. That is, a copy number aberration in a read can be viewed by detection method 400 as a variation in the microsatellite length. Variations in microsatellite length not caused by microsatellite instability can increase the number of false positives of detection method 400.

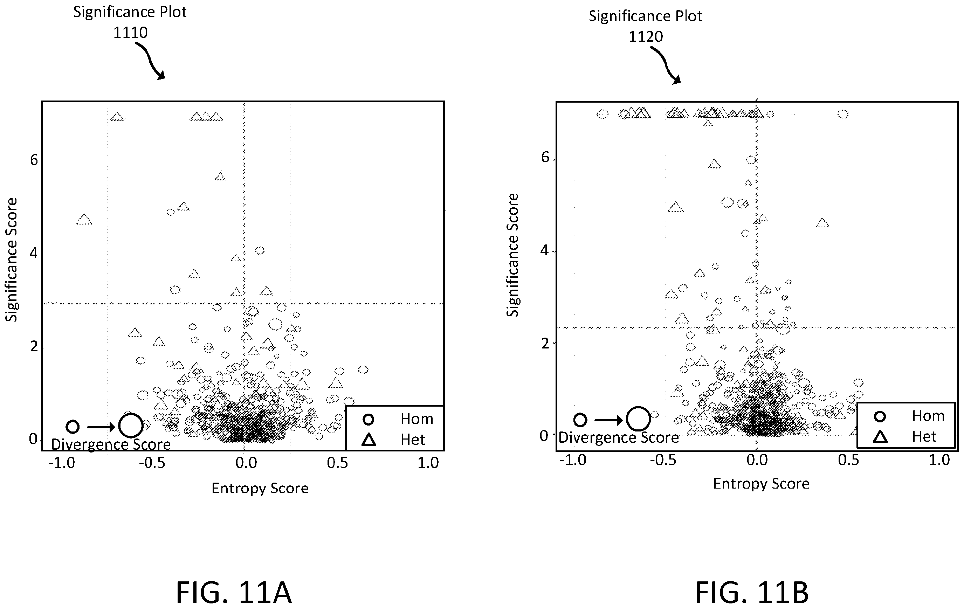

To illustrate this, FIGS. 11A-11B are significance plots of samples not including microsatellite instability but including markers with high significance scores, high divergence scores, and low entropy scores. Significance plot 1110 illustrates a sample using both gDNA and cfDNA and significance plot 1120 illustrates a sample using cfDNA. In these examples, some of the markers may be significant markers even when not including MSI signatures. Notably, many heterozygous markers (i.e., triangle shapes) are markers that have high significance and divergence scores that may be determined as significant markers.

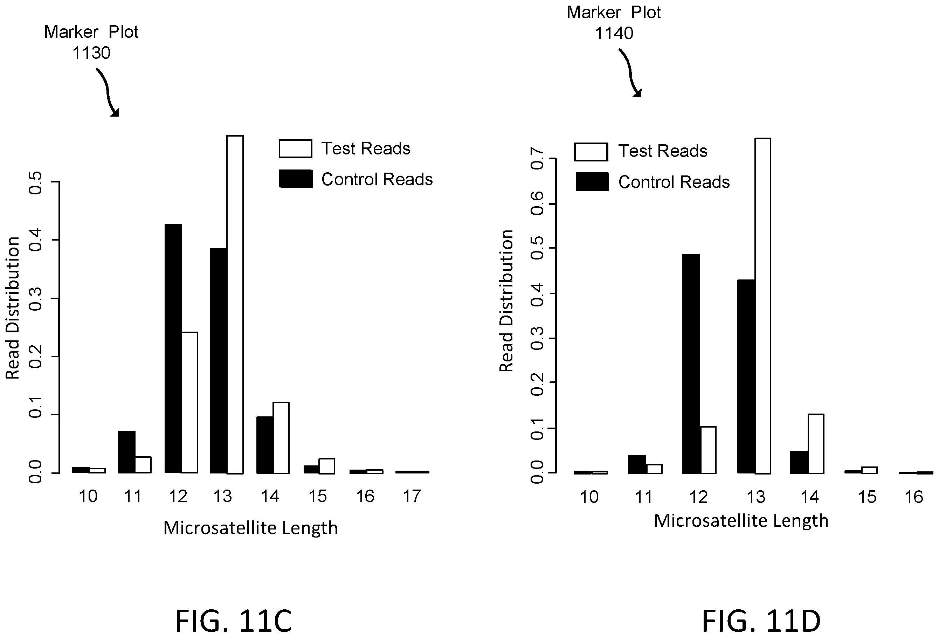

FIGS. 11C-11D are marker plots for a single heterozygous marker with a high significance and divergence score. In this example, an error correction model has not been applied to the markers. FIG. 11C is a marker plot 1130 showing data for a heterozygous marker from significance plot 1110, according to one example embodiment, according to one example embodiment. FIG. 11D is a marker plot 1140 showing data for a heterozygous marker from significance plot 1120. In the heterozygous markers, the probability distribution for microsatellite length is greater in the control reads than the test reads for both cfDNA and the combination of gDNA and cfDNA. The increased microsatellite length distribution in control reads over test reads is indicative of copy number aberration rather than MSI. In some cases, these markers may be incorrectly determined to be significant markers and may influence correct determination of the instability score for the sample. Accordingly, in some example embodiments, MSI detection method 400 can filter heterozygous reads before determining marker significance to increase specificity of MSI detection.

### VII. EXAMPLES

### VII.A LUNG

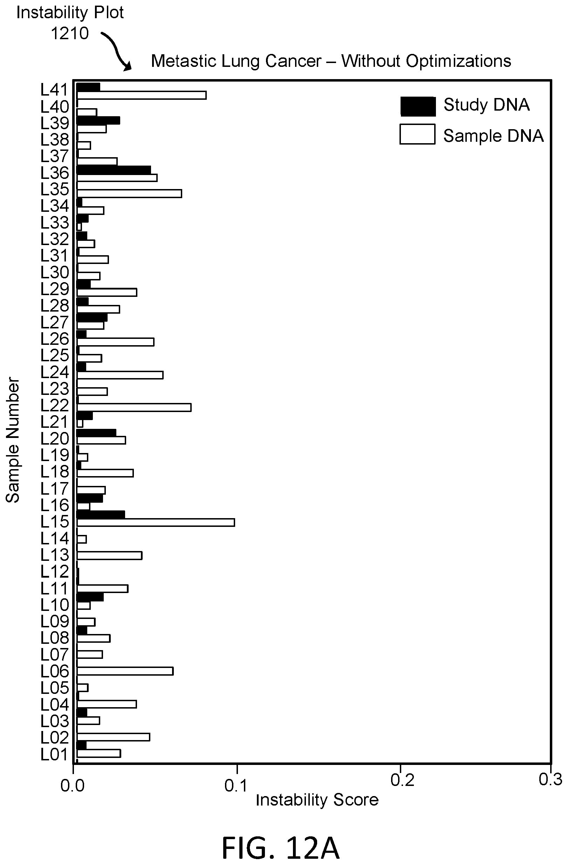

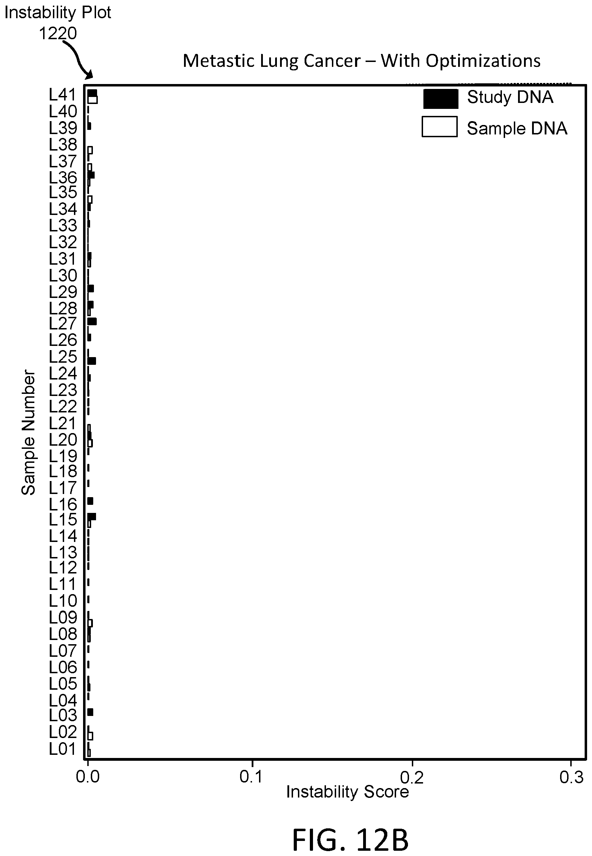

FIG. 12A is an instability plot 1210 of metastatic lung samples using a prior art method, according to one example embodiment. FIG. 12B is an instability plot 1220 metastatic lung samples using detection method 400, according to one example embodiment. Here, detection method 400 improves the sensitivity in MSI detection over the prior art method.

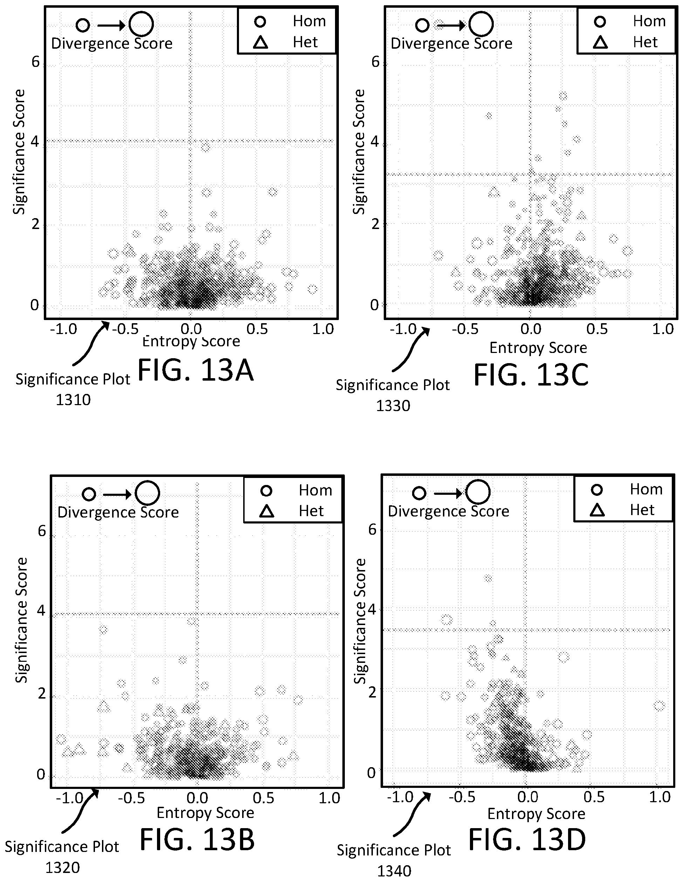

FIGS. 13A-13D are significance plots 1310-1340 of metastatic lung samples not including microsatellite instability. The samples are all from sample DNA in FIG. 13B. Significance plots 1310 and 1320 illustrate samples using cfDNA and gDNA and significance plot 1330 and 1340 illustrate a sample using cfDNA. The illustrated examples are for samples shown to have a high MSI score using a method known in the art and a low MSI score using method 400.

### VII.B BREAST

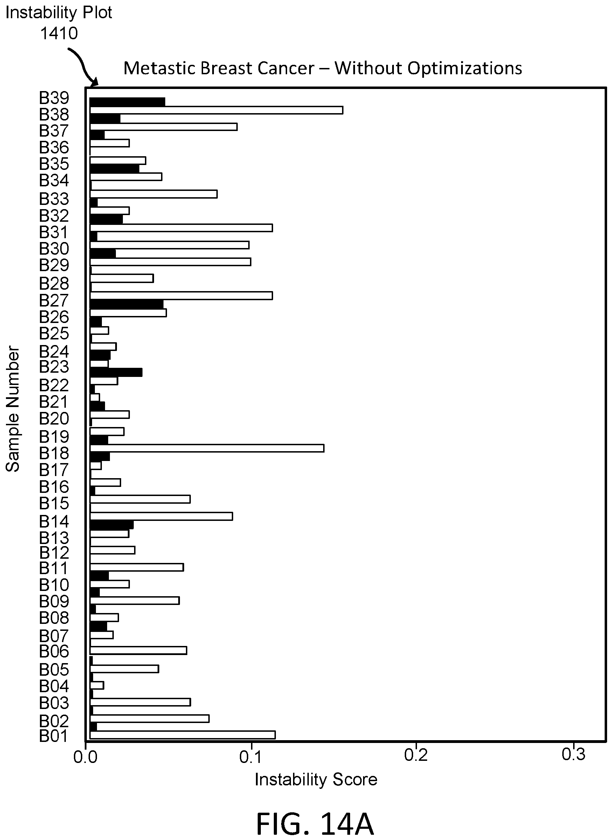

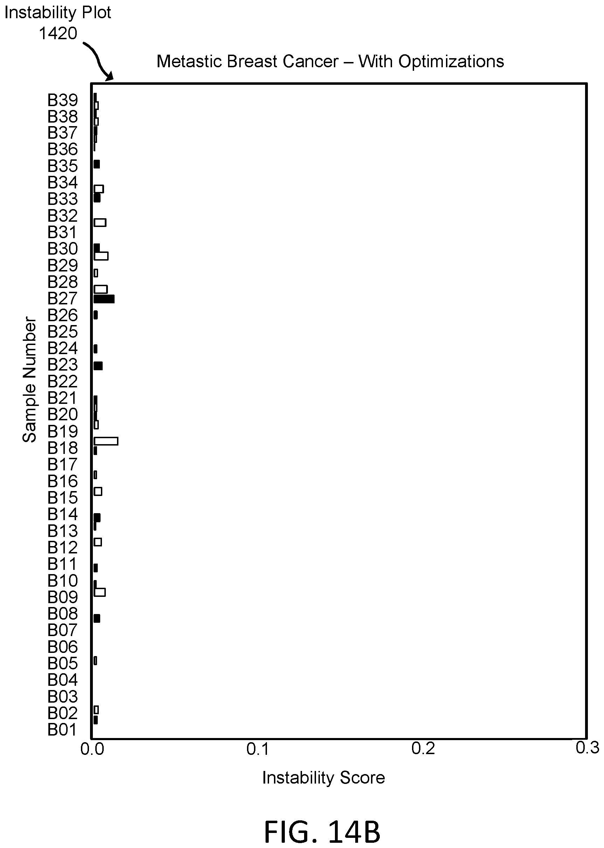

FIG. 14A is an instability plot 1410 of metastatic breast samples using a prior art method, according to one example embodiment. FIG. 14B is an instability plot 1420 metastatic breast samples using detection method 400, according to one example embodiment. Here, detection method 400 improves the sensitivity in MSI detection over the prior art method.

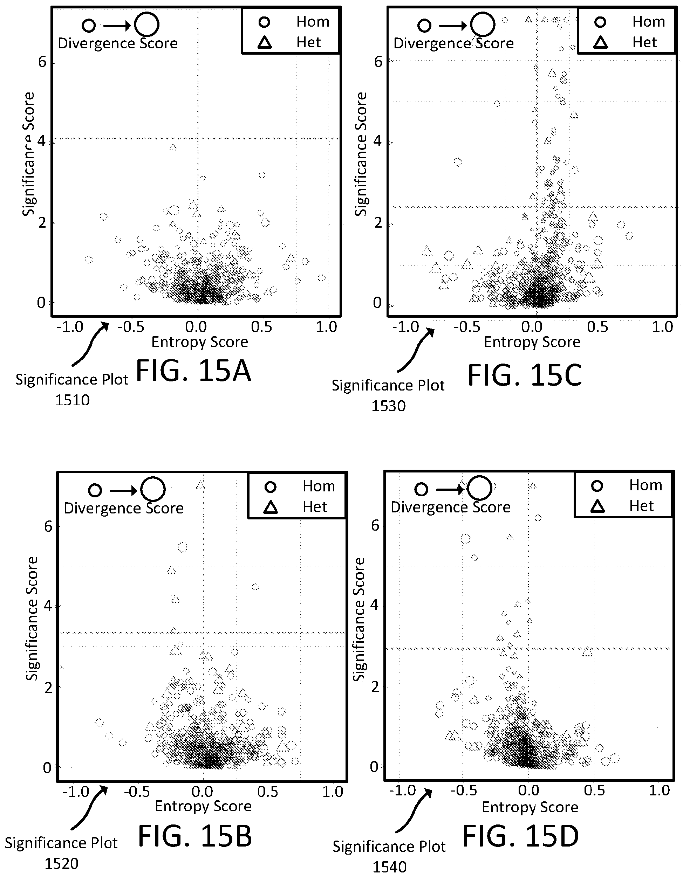

FIGS. 15A-15D are significance plots of metastatic breast samples not including microsatellite instability. The samples are all from the sample DNA in FIG. 14B. Significance plots 1510 and 1520 illustrate samples using gDNA and cfDNA and significance plot 1530 and 1540 illustrate a sample using cfDNA. The illustrated examples are for samples shown to have a high MSI score using a method known in the art and a low MSI score using method 400.

### VII.C PROSTATE

FIG. 5A is an instability plot 510 of metastatic prostate samples using a prior art method, according to one example embodiment. FIG. 5B is an instability plot 520 metastatic prostate samples using detection method 400, according to one example embodiment. Here, detection method 400 improves the sensitivity in MSI detection over the prior art method.

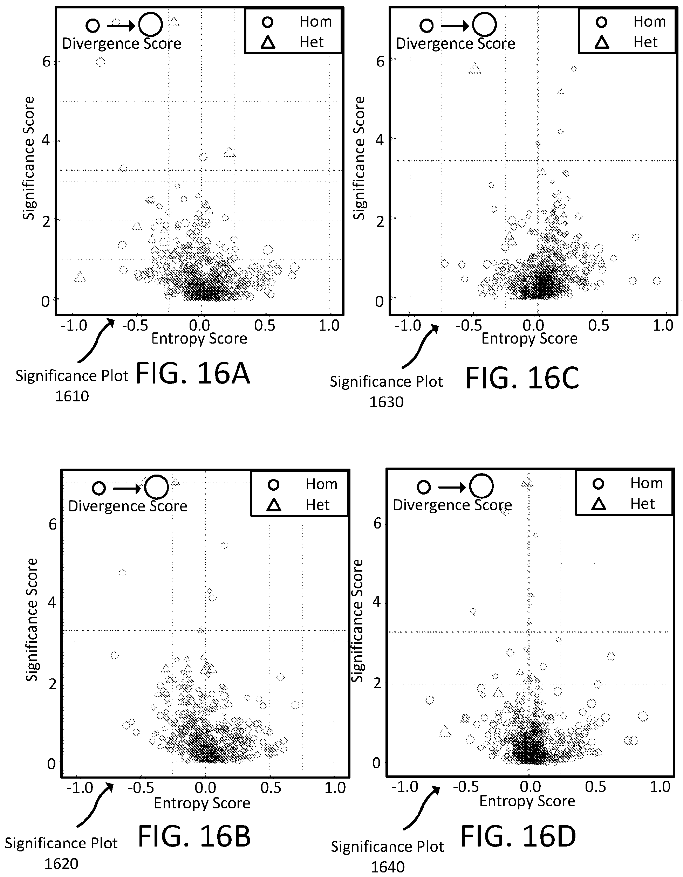

FIGS. 16A-16D are significance plots of metastatic prostate samples not including microsatellite instability. The samples are all from the sample DNA in FIG. 5B. Significance plots 1610 and 1620 illustrate samples using gDNA and cfDNA and significance plot 1630 and 1640 illustrate a sample using cfDNA. The illustrated examples are for samples shown to have a high MSI score using a method known in the art and a low MSI score using method 400.

### VII. ADDITIONAL CONSIDERATIONS

The foregoing description of the embodiments of the invention has been presented for the purpose of illustration; it is not intended to be exhaustive or to limit the invention to the precise forms disclosed. Persons skilled in the relevant art can appreciate that many modifications and variations are possible in light of the above disclosure.

Some portions of this description describe the embodiments of the invention in terms of algorithms and symbolic representations of operations on information. These algorithmic descriptions and representations are commonly used by those skilled in the data processing arts to convey the substance of their work effectively to others skilled in the art. These operations, while described functionally, computationally, or logically, are understood to be implemented by computer programs or equivalent electrical circuits, microcode, or the like. Furthermore, it has also proven convenient at times, to refer to these arrangements of operations as modules, without loss of generality. The described operations and their associated modules may be embodied in software, firmware, hardware, or any combinations thereof.

Any of the steps, operations, or processes described herein may be performed or implemented with one or more hardware or software modules, alone or in combination with other devices. In one embodiment, a software module is implemented with a computer program product including a computer-readable non-transitory medium containing computer program code, which can be executed by a computer processor for performing any or all of the steps, operations, or processes described.

Aspects of the disclosure may also relate to a product that is produced by a computing process described herein. Such a product may include information resulting from a computing process, where the information is stored on a non-transitory, tangible computer readable storage medium and may include any embodiment of a computer program product or other data combination described herein.

Finally, the language used in the specification has been principally selected for readability and instructional purposes, and it may not have been selected to delineate or circumscribe the inventive subject matter. It is therefore intended that the scope of the invention be limited not by this detailed description, but rather by any claims that issue on an application based hereon. Accordingly, the disclosure of the embodiments of the invention is intended to be illustrative, but not limiting, of the scope of the invention, which is set forth in the following claims.

## Claims

1. A computer-implemented method (400) for detecting a likelihood of cancer in an individual based on microsatellite instability (MSI), the method comprising:
accessing (410) a plurality of test reads and a plurality of control reads associated with a sample;
selecting (420) a plurality of markers from the test reads and the control reads, each marker identifying a set of nucleotides from the reads, the markers known to be associated with microsatellite instability in cancer;
filtering (430) the plurality of markers such that the specificity of determining an instability score increases; the filtering comprising removing heterozygous reads from sample reads such that determining marker significance and calculating instability scores uses only homozygous reads,
for each marker, calculating (440) a marker significance indicating the significance and viability for the marker in detecting MSI,
the viability representing a level of similarity between characteristics of test reads and control reads of the marker, and
the significance representing the statistical significance of a length variation of a repeated subset of nucleotides in the set of nucleotides in test reads of the marker relative to the length variation for the same repeated subset of nucleotides in the control reads of the marker;
wherein calculating the marker significance includes calculating marker scores based on the sample reads, the marker scores including a significance score and a viability score,
wherein calculating the significance score comprises quantifying the statistical significance of the variation in the microsatellite length distribution of test reads relative to control reads,
wherein calculating the viability score comprises determining a high viability score above a threshold viability score if the number of test reads and control reads is the same, and a low viability score below the threshold viability score for that marker, if there is a difference between the number of test reads and the number of control reads for a marker,
determining an instability score (450) for the sample based on the calculated marker significance scores, the instability score being a ratio of significant to insignificant markers representing a likelihood that the sample contains MSI and detecting a likelihood of cancer based on the likelihood that the sample contains a MSI;
wherein determining an instability score comprises
- when the viability score for the marker is below the threshold viability score, not using the marker to calculate the instability score for the sample and when the viability score for the marker is above the threshold viability score, using the marker to calculate the instability score for the sample and
- by comparing the significance score to a significance threshold, determining, for use in the instability score, if the marker is a significant marker or an insignificant marker.

2. The method of claim 1, wherein determining the marker significance further comprises:
calculating an entropy score representing the entropy of each marker, the entropy score a measure of a difference in entropy of the marker between the test reads and the control reads, where entropy is the average uncertainty in the series of nucleotides in the set of nucleotides;
optionally, wherein the difference in entropy of the marker is the entropy of the test read less the entropy of the control read; and/or
optionally, wherein determining the marker significance including calculating an entropy score further comprises:
evaluating the difference in entropy for each marker, wherein markers with the difference in entropy indicating the test read is more disordered than the control read being significant markers.

3. The method of claim 1 or 2, wherein determining the marker significance further comprises:
calculating a divergence score representing the relative entropy for the marker, the divergence score being a measure of a relative difference in expected observation of the length variation between test reads and control reads.

4. The method of claim 3, wherein determining the marker significance includ-
ing calculating said divergence score further comprises:
comparing the divergence score for the marker to a threshold divergence score, the markers with the divergence score greater than threshold divergence score being significant markers; and/or
wherein the divergence score is the Jenson-Shannon divergence between the test reads and the control reads.

5. The method of claim 1, wherein determining the marker significance including calculating said significance score further comprises:
applying a significance test to the significance score for each marker, the markers passing the significance test being significant markers.

6. The method of claim 5, wherein the significance test is the Benjamini-Hochberg correction; and/or
wherein the significance test is a method for detecting false discovery rates.

7. The method of claim 6, wherein determining the marker significance comprises:
applying an error correction model to a set of measurement errors of the test reads, the error correction model determining and correcting a characteristic of the test reads, a characteristic of the test reads including any of bag size, length distribution, duplex rate or sequence depth;
optionally, wherein the viability score quantifies the similarities between the characteristic in the test reads and the control reads; and/or
optionally, wherein the error correction model is any of: unique molecular index correction, duplex correction, stitching, or positional error correction.

8. The method of any of claims 1 to 7, wherein each marker of the plurality of markers has at least a threshold read depth of test reads and control reads; and/or
wherein the test reads and the control reads are obtained from cell-free nucleic acid; and/or
wherein the test reads and the control reads are obtained from a sample previously known not to include cancer cells; and/or
wherein the control reads are obtained from a secondary sample previously known not to include microsatellite instability.

9. A system comprising one or more processors and one or more memories storing computer instructions for detecting a likelihood of cancer in an individual based on microsatellite instability (MSI), the instructions when executed by the one or more processors causing the processor to perform steps including:
accessing (410) a plurality of test reads and a plurality of control reads associated with a sample;
selecting (420) a plurality of markers from the test reads and the control reads, each marker identifying a set of nucleotides from the reads, the markers known to be associated with microsatellite instability in cancer;
filtering (430) the plurality of markers such that the specificity of determining an instability score (450) increases; the filtering comprising removing heterozygous reads from sample reads such that determining marker significance and calculating instability scores uses only homozygous reads,
for each marker, calculating a marker significance indicating the significance and viability for the marker in detecting MSI,
the viability representing a level of similarity between characteristics of test reads and control reads of the marker, and
the significance representing the statistical significance of a length variation of a repeated subset of nucleotides in the set of nucleotides in test reads of the marker relative to the length variation for the same repeated subset of nucleotides in the control reads of the marker;
wherein calculating the marker significance includes calculating marker scores based on the sample reads, the marker scores including a significance score and a viability score,
wherein calculating the significance score comprises quantifying the statistical significance of the variation in the microsatellite length distribution of test reads relative to control reads,
wherein calculating the viability score comprises determining a high viability score above a threshold viability score if the number of test reads and control reads is the same, and a low viability score below the threshold viability score for that marker, if there is a difference between the number of test reads and the number of control reads for a marker,
determining (450) an instability score for the sample based on the calculated marker significance scores, the instability score a ratio of significant to insignificant markers representing a likelihood that the sample contains MSI and detecting a likelihood of cancer based on the likelihood that the sample contains a MSI;
wherein determining an instability score comprises
- when the viability score for the marker is below the threshold viability score, not using the marker to calculate the instability score for the sample and when the viability score for the marker is above the threshold viability score, using the marker to calculate the instability score for the sample and
- by comparing the significance score to a significance threshold, determining, for use in the instability score, if the marker is a significant marker or an insignificant marker.

10. The system of claim 9, wherein determining the marker significance further causes the one or more processors to perform steps including:
calculating an entropy score representing the entropy of each marker, the entropy score a measure of a difference in entropy of the marker between the test reads and the control reads, where entropy is the average uncertainty in the series of nucleotides in the set of nucleotides;
optionally, wherein the difference in entropy of the marker is the entropy of the test read less the entropy of the control read.

11. The system of claim 10, wherein determining the marker significance including calculating said entropy score further causes the one or more processors to perform steps including:
evaluating the difference in entropy for each marker, wherein markers with the difference in entropy indicating the test read is more disordered than the control read being significant markers.

12. The system of any of claims 9 to 11, wherein determining the marker significance further causes the one or more processors to perform steps including:
calculating a divergence score representing the relative entropy for the marker, the divergence score a measure of a relative difference in expected observation of the length variation between test reads and control reads.

13. The system of claim 12, wherein determining the marker significance including calculating said divergence score further causes the one or more processors to perform steps including:
comparing the divergence score for the marker to a threshold divergence score, the markers with the divergence score greater than threshold divergence score being significant markers and/or
wherein the divergence score is the Jenson-Shannon divergence between the test reads and the control reads.

14. The system of claim 9, wherein determining the marker significance including calculating significance score further causes the one or more processors to perform steps including:
applying a significance test to the significance score for each marker, the markers passing the significance test being significant markers;
optionally, wherein the significance test is the Benjamini-Hochberg correction; and/or
optionally, wherein the significance test is a method for detecting false discovery rates.

15. The system of claim 14, wherein determining the marker significance further causes the one or more processors to perform steps including:
applying an error correction model to a set of measurement errors of the test reads, the error correction model determining and correcting a characteristic of the test reads, a characteristic of the test reads including any of bag size, length distribution, duplex rate or sequence depth;
optionally, wherein the viability score quantifies the similarities between the characteristic in the test reads and the control reads; and/or
optionally, wherein the error correction model is any of: unique molecular index correction, duplex correction, stitching, or positional error correction.

16. The system of any of claims 9 to 15, wherein each marker of the plurality of markers has at least a threshold read depth of test reads and control reads; and/or
wherein the test reads and the control reads are obtained from cell-free nucleic acid; and/or
wherein the test reads and the control reads are obtained from a sample previously known not to include cancer cells; and/or
wherein the control reads are obtained from a secondary sample previously known not to include microsatellite instability.

## Patentansprüche

1. Computer-implementiertes Verfahren (400) zum Erkennen einer Wahrscheinlichkeit von Krebs in einem Individuum auf der Grundlage von Mikrosatelliteninstabilität (MSI), wobei das Verfahren umfasst:
Zugreifen (410) auf eine Vielzahl von Testlesungen und eine Vielzahl von Kontrolllesungen, die mit einer Probe verbunden sind;
Auswählen (420) einer Vielzahl von Markern aus den Testlesungen und den Kontrolllesungen, wobei jeder Marker einen Satz von Nukleotiden aus den Lesungen identifiziert, wobei die Marker bekanntermaßen mit Mikrosatelliteninstabilität bei Krebs assoziiert sind;
Filtern (430) der Vielzahl von Markern, so dass die Spezifität der Bestimmung eines Instabilitätswertes zunimmt; wobei das Filtern das Entfernen heterozygoter Lesungen aus den Probenlesungen umfasst, so dass die Bestimmung der Markersignifikanz und die Berechnung der Instabilitätswertes nur homozygote Lesungen verwendet,
für jeden Marker die Berechnung (440) einer Markersignifikanz, die die Signifikanz und Lebensfähigkeit des Markers bei der Erkennung von MSI angibt, die Viabilität, die den Grad der Ähnlichkeit zwischen den Merkmalen der Testlesungen und der Kontrolllesungen des Markers darstellt, und
wobei die Signifikanz die statistische Signifikanz einer Längenvariation einer sich wiederholenden Untergruppe von Nukleotiden in dem Satz von Nukleotiden in Testlesungen des Markers relativ zu der Längenvariation für dieselbe sich wiederholende Untergruppe von Nukleotiden in den Kontrolllesungen des Markers darstellt;
wobei das Berechnen der Markersignifikanz das Berechnen von Markerwertes auf der Grundlage der Probenlesungen umfasst, wobei die Markerwertes einen Signifikanzwert und einen Viabilitätswert umfassen,
wobei die Berechnung des Signifikanzwertes die Quantifizierung der statistischen Signifikanz der Variation in der Mikrosatelliten-Längenverteilung der Testlesungen im Vergleich zu den Kontrolllesungen umfasst,
wobei das Berechnen des Viabilitätswertes das Bestimmen eines hohen Viabilitätswertes oberhalb eines Schwellen-Viabilitätswertes umfasst, wenn die Anzahl der Testlesungen und der Kontrolllesungen gleich ist, und eines niedrigen Viabilitätswertes unterhalb des Schwellen-Viabilitätswertes für diesen Marker, wenn es einen Unterschied zwischen der Anzahl der Testlesungen und der Anzahl der Kontrolllesungen für einen Marker gibt,
Bestimmen eines Instabilitätswertes (450) für die Probe auf der Grundlage der berechneten Markersignifikanzwerts, wobei der Instabilitätswert ein Verhältnis von signifikanten zu insignifikanten Markern ist, das eine Wahrscheinlichkeit darstellt, dass die Probe MSI enthält, und Erfassen einer Krebswahrscheinlichkeit auf der Grundlage der Wahrscheinlichkeit, dass die Probe eine MSI enthält;
wobei die Bestimmung einer Instabilitätsbewertung Folgendes umfasst
- wenn der Lebensfähigkeitswert für den Marker unter dem Schwellenwert für die Lebensfähigkeit liegt, wird der Marker nicht zur Berechnung des Instabilitätswertes für die Probe verwendet, und wenn der Lebensfähigkeitswert für den Marker über dem Schwellenwert für die Lebensfähigkeit liegt, wird der Marker zur Berechnung des Instabilitätswertes für die Probe verwendet, und
- durch Vergleich des Signifikanzwertes mit einer Signifikanzschwelle zur Verwendung im Instabilitätswert bestimmen, ob der Marker ein signifikanter Marker oder ein nicht signifikanter Marker ist.

2. Verfahren nach Anspruch 1, wobei die Bestimmung der Signifikanz des Markers weiterhin umfasst:
Berechnen eines Entropiewertes, der die Entropie jedes Markers darstellt, wobei der Entropiewert ein Maß für den Unterschied in der Entropie des Markers zwischen den Testlesungen und den Kontrolllesungen ist, wobei die Entropie die durchschnittliche Unsicherheit in der Reihe von Nukleotiden in der Gruppe von Nukleotiden ist;
optional, wobei der Unterschied in der Entropie des Markers die Entropie der Testablesung abzüglich der Entropie der Kontrollablesung ist; und/oder
optional, wobei die Bestimmung der Markersignifikanz einschließlich der Berechnung eines Entropiewertes weiterhin umfasst:
Auswerten der Differenz in der Entropie für jeden Marker, wobei Marker mit der Differenz in der Entropie, die anzeigt, dass die Testablesung stärker ungeordnet ist als die Kontrollablesung, signifikante Marker sind.

3. Verfahren nach Anspruch 1 oder 2, wobei die Bestimmung der Signifikanz des Markers weiterhin umfasst:
Berechnen eines Divergenzwertes, der die relative Entropie für den Marker darstellt, wobei der Divergenzwert ein Maß für den relativen Unterschied bei der erwarteten Beobachtung der Längenvariation zwischen Testlesungen und Kontrolllesungen ist.

4. Verfahren nach Anspruch 3, wobei die Bestimmung der Markersignifikanz einschließlich der Berechnung des Divergenzwertes ferner Folgendes umfasst:
Vergleichen des Divergenzwertes für den Marker mit einem Schwellenwert für den Divergenzwert, wobei die Marker mit dem Divergenzwert, der größer als der Schwellenwert für den Divergenzwert ist, signifikante Marker sind;
und/oder
wobei der Divergenzwert die Jenson-Shannon-Divergenz zwischen den Testlesungen und den Kontrolllesungen ist.

5. Verfahren nach Anspruch 1, wobei die Bestimmung der Markersignifikanz einschließlich der Berechnung der Signifikanz-Punktzahl weiterhin umfasst:
Anwendung eines Signifikanztests auf den Signifikanzwert für jeden Marker, wobei die Marker, die den Signifikanztest bestehen, signifikante Marker sind.

6. Verfahren nach Anspruch 5, wobei der Signifikanztest die Benjamini-Hochberg-Korrektur ist; und/oder
wobei der Signifikanztest ein Verfahren zur Erkennung von Falschentdeckungsraten ist.

7. Verfahren nach Anspruch 6, wobei die Bestimmung der Markersignifikanz umfasst:
Anwenden eines Fehlerkorrekturmodells auf einen Satz von Messfehlern der Testleseproben, wobei das Fehlerkorrekturmodell eine Eigenschaft der Testleseproben bestimmt und korrigiert, wobei eine Eigenschaft der Testleseproben irgendetwas von Beutelgröße, Längenverteilung, Duplexrate oder Sequenztiefe umfasst;
optional, wobei die Viabilitätsbewertung die Ähnlichkeiten zwischen dem Merkmal in den Testlesungen und den Kontrolllesungen quantifiziert; und/oder
optional, wobei das Fehlerkorrekturmodell eines der folgenden ist: eindeutige Molekularindexkorrektur, Duplexkorrektur, Stitching oder Positionsfehlerkorrektur.

8. Verfahren nach einem der Ansprüche 1 bis 7, wobei jeder Marker der Vielzahl von Markern mindestens eine Schwellenwert-Lesetiefe von Testlesungen und Kontrolllesungen aufweist; und/oder
wobei die Testlesungen und die Kontrolllesungen aus zellfreier Nukleinsäure gewonnen werden; und/oder
wobei die Testlesungen und die Kontrolllesungen aus einer Probe gewonnen werden, von der zuvor bekannt war, dass sie keine Krebszellen enthält;
und/oder
wobei die Kontrolllesungen aus einer Sekundärprobe gewonnen werden, von der zuvor bekannt war, dass sie keine Mikrosatelliteninstabilität enthält.

9. System mit einem oder mehreren Prozessoren und einem oder mehreren Speichern, die Computerbefehle zum Erkennen einer Wahrscheinlichkeit von Krebs in einem Individuum auf der Grundlage von Mikrosatelliteninstabilität (MSI) speichern, wobei die Befehle, wenn sie von dem einen oder den mehreren Prozessoren ausgeführt werden, den Prozessor veranlassen, Schritte auszuführen, die Folgendes umfassen:
Zugreifen (410) auf eine Vielzahl von Testlesungen und eine Vielzahl von Kontrolllesungen, die mit einer Probe verbunden sind;
Auswählen (420) einer Vielzahl von Markern aus den Testlesungen und den Kontrolllesungen, wobei jeder Marker einen Satz von Nukleotiden aus den Lesungen identifiziert, wobei die Marker bekanntermaßen mit Mikrosatelliteninstabilität bei Krebs assoziiert sind;
Filtern (430) der Vielzahl von Markern, so dass die Spezifität der Bestimmung eines Instabilitätswertes (450) zunimmt; wobei das Filtern das Entfernen heterozygoter Lesungen aus den Probenlesungen umfasst, so dass die Bestimmung der Markersignifikanz und die Berechnung der Instabilitätswertes nur homozygote Lesungen verwendet,
für jeden Marker, wobei eine Markersignifikanz berechnet wird, die die Signifikanz und Lebensfähigkeit des Markers beim Nachweis von MSI angibt, die Viabilität, die den Grad der Ähnlichkeit zwischen den Merkmalen der Testlesungen und der Kontrolllesungen des Markers darstellt, und
wobei die Signifikanz die statistische Signifikanz einer Längenvariation einer sich wiederholenden Untergruppe von Nukleotiden in dem Satz von Nukleotiden in Testlesungen des Markers relativ zu der Längenvariation für dieselbe sich wiederholende Untergruppe von Nukleotiden in den Kontrolllesungen des Markers darstellt;
wobei das Berechnen der Markersignifikanz das Berechnen von Markerwertes auf der Grundlage der Probenlesungen umfasst, wobei die Markerwertes einen Signifikanzwert und einen Viabilitätswert umfassen,
wobei die Berechnung des Signifikanzwertes die Quantifizierung der statistischen Signifikanz der Variation in der Mikrosatelliten-Längenverteilung der Testlesungen im Vergleich zu den Kontrolllesungen umfasst,
wobei das Berechnen des Viabilitätswertes das Bestimmen eines hohen Viabilitätswertes oberhalb eines Schwellen-Viabilitätswertes umfasst, wenn die Anzahl der Testlesungen und der Kontrolllesungen gleich ist, und eines niedrigen Viabilitätswertes unterhalb des Schwellen-Viabilitätswertes für diesen Marker, wenn es einen Unterschied zwischen der Anzahl der Testlesungen und der Anzahl der Kontrolllesungen für einen Marker gibt,
Bestimmen (450) eines Instabilitätswertes für die Probe auf der Grundlage der berechneten Markersignifikanzwerts, wobei der Instabilitätswert ein Verhältnis von signifikanten zu insignifikanten Markern ist, das eine Wahrscheinlichkeit darstellt, dass die Probe MSI enthält, und Erfassen einer Krebswahrscheinlichkeit auf der Grundlage der Wahrscheinlichkeit, dass die Probe eine MSI enthält;
wobei die Bestimmung einer Instabilitätsbewertung Folgendes umfasst
- wenn der Lebensfähigkeitswert für den Marker unter dem Schwellenwert für die Lebensfähigkeit liegt, wird der Marker nicht zur Berechnung des Instabilitätswertes für die Probe verwendet, und wenn der Lebensfähigkeitswert für den Marker über dem Schwellenwert für die Lebensfähigkeit liegt, wird der Marker zur Berechnung des Instabilitätswertes für die Probe verwendet, und
- durch Vergleich des Signifikanzwerts mit einem Signifikanzschwellenwert zur Verwendung im Instabilitätswert bestimmen, ob der Marker ein signifikanter Marker oder ein nicht signifikanter Marker ist.

10. System nach Anspruch 9, wobei das Bestimmen der Markersignifikanz ferner den einen oder die mehreren Prozessoren veranlasst, Schritte auszuführen, die Folgendes umfassen:
Berechnen eines Entropiewertes, der die Entropie jedes Markers darstellt, wobei der Entropiewert ein Maß für den Unterschied in der Entropie des Markers zwischen den Testlesungen und den Kontrolllesungen ist, wobei die Entropie die durchschnittliche Unsicherheit in der Reihe von Nukleotiden in der Gruppe von Nukleotiden ist;
optional, wobei der Unterschied in der Entropie des Markers die Entropie der Testablesung abzüglich der Entropie der Kontrollablesung ist.

11. System nach Anspruch 10, wobei das Bestimmen der Markersignifikanz einschließlich des Berechnens des Entropiewertes weiterhin den einen oder die mehreren Prozessoren veranlasst, Schritte auszuführen, die Folgendes umfassen:
Auswerten der Differenz in der Entropie für jeden Marker, wobei Marker mit der Differenz in der Entropie, die anzeigt, dass die Testablesung stärker ungeordnet ist als die Kontrollablesung, signifikante Marker sind.

12. System nach einem der Ansprüche 9 bis 11, wobei die Bestimmung der Markersignifikanz ferner den einen oder die mehreren Prozessoren veranlasst, Schritte auszuführen, die Folgendes umfassen:
Berechnen eines Divergenzwertes, der die relative Entropie für den Marker darstellt, wobei der Divergenzwert ein Maß für einen relativen Unterschied in der erwarteten Beobachtung der Längenvariation zwischen Testlesungen und Kontrolllesungen ist.

13. System nach Anspruch 12, wobei das Bestimmen der Markersignifikanz einschließlich des Berechnens des Divergenzwertes ferner den einen oder die mehreren Prozessoren veranlasst, Schritte auszuführen, die Folgendes umfassen:
Vergleichen des Divergenzwertes für den Marker mit einem Schwellenwert für den Divergenzwert, wobei die Marker mit dem Divergenzwert größer als der Schwellenwert für den Divergenzwert signifikante Marker sind und/oder wobei der Divergenzwert die Jenson-Shannon-Divergenz zwischen den Testlesungen und den Kontrolllesungen ist.

14. System nach Anspruch 9, wobei die Bestimmung der Markersignifikanz einschließlich der Berechnung des Signifikanzwerts weiterhin den einen oder die mehreren Prozessoren veranlasst, Schritte auszuführen, die Folgendes umfassen:
Anwendung eines Signifikanztests auf den Signifikanzwert für jeden Marker, wobei die Marker, die den Signifikanztest bestehen, signifikante Marker sind;
optional, wobei der Signifikanztest die Benjamini-Hochberg-Korrektur ist;
und/oder
optional, wobei der Signifikanztest ein Verfahren zur Erkennung von Falschentdeckungsraten ist.

15. System nach Anspruch 14, wobei das Bestimmen der Markersignifikanz ferner den einen oder die mehreren Prozessoren veranlasst, Schritte auszuführen, die Folgendes umfassen:
Anwenden eines Fehlerkorrekturmodells auf einen Satz von Messfehlern der Testleseproben, wobei das Fehlerkorrekturmodell eine Eigenschaft der Testleseproben bestimmt und korrigiert, wobei eine Eigenschaft der Testleseproben irgendetwas von Beutelgröße, Längenverteilung, Duplexrate oder Sequenztiefe umfasst;
optional, wobei die Viabilitätsbewertung die Ähnlichkeiten zwischen dem Merkmal in den Testlesungen und den Kontrolllesungen quantifiziert; und/oder
optional, wobei das Fehlerkorrekturmodell eines der folgenden ist: eindeutige Molekularindexkorrektur, Duplexkorrektur, Stitching oder Positionsfehlerkorrektur.

16. System nach einem der Ansprüche 9 bis 15, wobei jeder Marker der Vielzahl von Markern mindestens eine Schwellenwert-Lesetiefe von Testlesungen und Kontrolllesungen aufweist; und/oder
wobei die Testlesungen und die Kontrolllesungen aus zellfreier Nukleinsäure gewonnen werden; und/oder
wobei die Testlesungen und die Kontrolllesungen aus einer Probe gewonnen werden, von der zuvor bekannt war, dass sie keine Krebszellen enthält;
und/oder
wobei die Kontrolllesungen aus einer Sekundärprobe gewonnen werden, von der zuvor bekannt war, dass sie keine Mikrosatelliteninstabilität enthält.

## Revendications

1. Méthode mise en oeuvre par ordinateur (400) pour détecter une probabilité de cancer chez un individu sur la base de l'instabilité des microsatellites (MSI), la méthode comprenant:
accéder (410) à une pluralité de lectures de test et à une pluralité de lectures de contrôle associées à un échantillon;
sélection (420) d'une pluralité de marqueurs à partir des lectures de test et des lectures de contrôle, chaque marqueur identifiant un ensemble de nucléotides à partir des lectures, les marqueurs étant connus pour être associés à l'instabilité des microsatellites dans le cancer;
filtrer (430) la pluralité de marqueurs de sorte que la spécificité de la détermination d'un score d'instabilité augmente; le filtrage comprend l'élimination des lectures hétérozygotes des lectures de l'échantillon de sorte que la détermination de la signification du marqueur et le calcul des scores d'instabilité n'utilisent que des lectures homozygotes,
pour chaque marqueur, calculer (440) une signification du marqueur indiquant la signification et la viabilité du marqueur dans la détection des MSI, la viabilité représente un niveau de similitude entre les caractéristiques des lectures de test et des lectures de contrôle du marqueur, et
la signification représente la signification statistique d'une variation de longueur d'un sous-ensemble répété de nucléotides dans l'ensemble de nucléotides dans les lectures de test du marqueur par rapport à la variation de longueur pour le même sous-ensemble répété de nucléotides dans les lectures de contrôle du marqueur;
le calcul de l'importance du marqueur comprend le calcul des scores du marqueur sur la base des lectures de l'échantillon, les scores du marqueur comprenant un score d'importance et un score de viabilité,
le calcul du score de signification consiste à quantifier la signification statistique de la variation de la distribution de la longueur des microsatellites des lectures de test par rapport aux lectures de contrôle,
le calcul du score de viabilité consiste à déterminer un score de viabilité élevé supérieur à un score de viabilité seuil si le nombre de lectures de test et de contrôle est identique, et un score de viabilité faible inférieur au score de viabilité seuil pour ce marqueur, s'il y a une différence entre le nombre de lectures de test et le nombre de lectures de contrôle pour un marqueur, déterminer un score d'instabilité (450) pour l'échantillon sur la base des scores de signification des marqueurs calculés, le score d'instabilité étant un rapport entre les marqueurs significatifs et non significatifs représentant une probabilité que l'échantillon contienne un MSI et détecter une probabilité de cancer sur la base de la probabilité que l'échantillon contienne un MSI;
dans lequel la détermination d'un score d'instabilité comprend
- lorsque le score de viabilité du marqueur est inférieur au score de viabilité seuil, ne pas utiliser le marqueur pour calculer le score d'instabilité de l'échantillon et lorsque le score de viabilité du marqueur est supérieur au score de viabilité seuil, utiliser le marqueur pour calculer le score d'instabilité de l'échantillon et
- en comparant le score de signification à un seuil de signification, déterminer, pour l'utiliser dans le score d'instabilité, si le marqueur est un marqueur significatif ou un marqueur non significatif.

2. La méthode de la revendication 1, dans laquelle la détermination de la signification du marqueur comprend en outre:
calculer un score d'entropie représentant l'entropie de chaque marqueur, le score d'entropie étant une mesure de la différence d'entropie du marqueur entre les lectures de test et les lectures de contrôle, l'entropie étant l'incertitude moyenne dans la série de nucléotides de l'ensemble de nucléotides;
éventuellement, dans lequel la différence d'entropie du marqueur est l'entropie de la lecture de test moins l'entropie de la lecture de contrôle; et/ou éventuellement, dans lequel la détermination de la signification du marqueur, y compris le calcul d'un score d'entropie, comprend en outre:
évaluer la différence d'entropie pour chaque marqueur, les marqueurs dont la différence d'entropie indique que la lecture test est plus désordonnée que la lecture de contrôle étant des marqueurs significatifs.

3. La méthode de la revendication 1 ou 2, dans laquelle la détermination de la signification du marqueur comprend en outre:
calculer un score de divergence représentant l'entropie relative pour le marqueur, le score de divergence étant une mesure de la différence relative dans l'observation attendue de la variation de longueur entre les lectures d'essai et les lectures de contrôle.

4. La méthode de la revendication 3, dans laquelle la détermination de la signification du marqueur, y compris le calcul du score de divergence, comprend en outre:
comparer le score de divergence du marqueur à un score de divergence seuil, les marqueurs dont le score de divergence est supérieur au score de divergence seuil étant des marqueurs significatifs; et/ou
dans lequel le score de divergence est la divergence de Jenson-Shannon entre les lectures de test et les lectures de contrôle.

5. La méthode de la revendication 1, dans laquelle la détermination de la signification du marqueur, y compris le calcul de ce score de signification, comprend en outre:
appliquer un test de signification au score de signification pour chaque marqueur, les marqueurs qui passent le test de signification étant des marqueurs significatifs.

6. La méthode de la revendication 5, dans laquelle le test de signification est la correction de Benjamini-Hochberg; et/ou
le test de signification est une méthode de détection des taux de fausse découverte.

7. La méthode de la revendication 6, dans laquelle la détermination de la signification du marqueur comprend:
appliquer un modèle de correction d'erreur à un ensemble d'erreurs de mesure des lectures d'essai, le modèle de correction d'erreur déterminant et corrigeant une caractéristique des lectures d'essai, une caractéristique des lectures d'essai comprenant la taille du sac, la distribution de la longueur, le taux de duplex ou la profondeur de la séquence;
éventuellement, dans lequel le score de viabilité quantifie les similitudes entre la caractéristique dans les lectures de test et les lectures de contrôle;
et/ou
éventuellement, le modèle de correction d'erreur est l'un des modèles suivants: correction de l'indice moléculaire unique, correction duplex, assemblage ou correction d'erreur de position.

8. La méthode de l'une des revendications 1 à 7, dans laquelle chaque marqueur de la pluralité de marqueurs a au moins un seuil de profondeur de lecture des lectures de test et des lectures de contrôle; et/ou
dans lequel les lectures de test et les lectures de contrôle sont obtenues à partir d'acide nucléique acellulaire; et/ou
dans lequel les lectures de test et les lectures de contrôle sont obtenues à partir d'un échantillon dont on sait qu'il ne contient pas de cellules cancéreuses; et/ou
les lectures de contrôle sont obtenues à partir d'un échantillon secondaire dont on sait qu'il ne présente pas d'instabilité microsatellitaire.

9. Système comprenant un ou plusieurs processeurs et une ou plusieurs mémoires stockant des instructions informatiques pour détecter une probabilité de cancer chez un individu sur la base de l'instabilité des microsatellites (MSI), les instructions, lorsqu'elles sont exécutées par un ou plusieurs processeurs, amènent le processeur à effectuer les étapes suivantes:
accéder (410) à une pluralité de lectures de test et à une pluralité de lectures de contrôle associées à un échantillon;
sélection (420) d'une pluralité de marqueurs à partir des lectures de test et des lectures de contrôle, chaque marqueur identifiant un ensemble de nucléotides à partir des lectures, les marqueurs étant connus pour être associés à l'instabilité des microsatellites dans le cancer;
filtrer (430) la pluralité de marqueurs de sorte que la spécificité de la détermination d'un score d'instabilité (450) augmente; le filtrage comprend l'élimination des lectures hétérozygotes des lectures de l'échantillon de sorte que la détermination de la signification du marqueur et le calcul des scores d'instabilité n'utilisent que des lectures homozygotes,
pour chaque marqueur, le calcul de la signification du marqueur indiquant la signification et la viabilité du marqueur dans la détection des MSI,
la viabilité représente un niveau de similitude entre les caractéristiques des lectures de test et des lectures de contrôle du marqueur, et
la signification représente la signification statistique d'une variation de longueur d'un sous-ensemble répété de nucléotides dans l'ensemble de nucléotides dans les lectures de test du marqueur par rapport à la variation de longueur pour le même sous-ensemble répété de nucléotides dans les lectures de contrôle du marqueur;
le calcul de l'importance du marqueur comprend le calcul des scores du marqueur sur la base des lectures de l'échantillon, les scores du marqueur comprenant un score d'importance et un score de viabilité,
le calcul du score de signification consiste à quantifier la signification statistique de la variation de la distribution de la longueur des microsatellites des lectures de test par rapport aux lectures de contrôle,
le calcul du score de viabilité consiste à déterminer un score de viabilité élevé supérieur à un score de viabilité seuil si le nombre de lectures de test et de contrôle est identique, et un score de viabilité faible inférieur au score de viabilité seuil pour ce marqueur, s'il y a une différence entre le nombre de lectures de test et le nombre de lectures de contrôle pour un marqueur,
déterminer (450) un score d'instabilité pour l'échantillon sur la base des scores de signification des marqueurs calculés, le score d'instabilité étant un rapport entre les marqueurs significatifs et les marqueurs non significatifs représentant une probabilité que l'échantillon contienne une MSI, et détecter une probabilité de cancer sur la base de la probabilité que l'échantillon contienne une MSI;
dans lequel la détermination d'un score d'instabilité comprend
- lorsque le score de viabilité du marqueur est inférieur au score de viabilité seuil, ne pas utiliser le marqueur pour calculer le score d'instabilité de l'échantillon et lorsque le score de viabilité du marqueur est supérieur au score de viabilité seuil, utiliser le marqueur pour calculer le score d'instabilité de l'échantillon et
- en comparant le score de signification à un seuil de signification, déterminer, pour l'utiliser dans le score d'instabilité, si le marqueur est un marqueur significatif ou un marqueur non significatif.

10. Le système de la revendication 9, dans lequel la détermination de l'importance du marqueur amène en outre le ou les processeurs à exécuter les étapes suivantes:
calculer un score d'entropie représentant l'entropie de chaque marqueur, le score d'entropie étant une mesure de la différence d'entropie du marqueur entre les lectures de test et les lectures de contrôle, l'entropie étant l'incertitude moyenne dans la série de nucléotides de l'ensemble de nucléotides;
éventuellement, la différence d'entropie du marqueur étant l'entropie de la lecture de test moins l'entropie de la lecture de contrôle.

11. Le système de la revendication 10, dans lequel la détermination de l'importance du marqueur, y compris le calcul du score d'entropie, amène un ou plusieurs processeurs à exécuter les étapes suivantes:
évaluer la différence d'entropie pour chaque marqueur, les marqueurs dont la différence d'entropie indique que la lecture test est plus désordonnée que la lecture de contrôle étant des marqueurs significatifs.

12. Le système de l'une des revendications 9 à 11, dans lequel la détermination de l'importance du marqueur amène en outre le ou les processeurs à exécuter les étapes suivantes:
calculer un score de divergence représentant l'entropie relative pour le marqueur, le score de divergence étant une mesure de la différence relative dans l'observation attendue de la variation de longueur entre les lectures d'essai et les lectures de contrôle.

13. Le système de la revendication 12, dans lequel la détermination de l'importance du marqueur, y compris le calcul du score de divergence, amène le ou les processeurs à exécuter les étapes suivantes:
comparer le score de divergence du marqueur à un score de divergence seuil, les marqueurs dont le score de divergence est supérieur au score de divergence seuil étant des marqueurs significatifs et/ou
dans lequel le score de divergence est la divergence de Jenson-Shannon entre les lectures de test et les lectures de contrôle.

14. Le système de la revendication 9, dans lequel la détermination de l'importance du marqueur, y compris le calcul du score d'importance, amène l'un ou plusieurs processeurs à exécuter les étapes suivantes:
appliquer un test de signification au score de signification pour chaque marqueur, les marqueurs qui passent le test de signification étant des marqueurs significatifs;
éventuellement, dans lequel le test de signification est la correction de Benjamini-Hochberg; et/ou
éventuellement, dans lequel le test de signification est une méthode de détection des taux de fausse découverte.

15. Le système de la revendication 14, dans lequel la détermination de l'importance du marqueur amène en outre le ou les processeurs à exécuter les étapes suivantes:
appliquer un modèle de correction d'erreur à un ensemble d'erreurs de mesure des lectures d'essai, le modèle de correction d'erreur déterminant et corrigeant une caractéristique des lectures d'essai, une caractéristique des lectures d'essai comprenant la taille du sac, la distribution de la longueur, le taux de duplex ou la profondeur de la séquence;
éventuellement, dans lequel le score de viabilité quantifie les similitudes entre la caractéristique dans les lectures de test et les lectures de contrôle;
et/ou
éventuellement, le modèle de correction d'erreur est l'un des modèles suivants: correction de l'indice moléculaire unique, correction duplex, assemblage ou correction d'erreur de position.

16. Le système de l'une des revendications 9 à 15, dans lequel chaque marqueur de la pluralité de marqueurs a au moins un seuil de profondeur de lecture des lectures de test et des lectures de contrôle; et/ou
dans lequel les lectures de test et les lectures de contrôle sont obtenues à partir d'acide nucléique acellulaire; et/ou
dans lequel les lectures de test et les lectures de contrôle sont obtenues à partir d'un échantillon dont on sait qu'il ne contient pas de cellules cancéreuses; et/ou
les lectures de contrôle sont obtenues à partir d'un échantillon secondaire dont on sait qu'il ne présente pas d'instabilité microsatellitaire.
